(19) Europäisches Patentamt
European Patent Office
Office européen des brevets

(11) EP 1 853 241 B1

(12) EUROPEAN PATENT SPECIFICATION

(45) Date of publication and mention of the grant of the patent:
**14.09.2011 Bulletin 2011/37**

(21) Application number: **06709910.1**

(22) Date of filing: **27.02.2006**

(51) Int Cl.:
*A61K 31/122* (2006.01) *A61P 25/16* (2006.01)

(86) International application number:
**PCT/GB2006/000684**

(87) International publication number:
**WO 2006/090177 (31.08.2006 Gazette 2006/35)**

(54) **USE OF 2-(2-NITRO-4-TRIFLUOROMETHYLBENZOYL)-1,3- CYCLOHEXANEDIONE IN THE TREATMENT OF PARKINSON'S DISEASE**

VERWENDUNG VON 2-(2-NITRO-4-TRIFLUORMETHYLBENZOYL)-1,3-CYCLOHEXANDION BEI DER BEHANDLUNG DER PARKINSON-KRANKHEIT

UTILISATION DE 2-(2-NITRO-4-TRIFLUOROMÉTHYLBENZOYL)-1,3- CYCLOHEXANEDIONE DANS LE TRAITEMENT DE LA MALADIE DE PARKINSON

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.02.2005 GB 0504103**

(43) Date of publication of application:
**14.11.2007 Bulletin 2007/46**

(73) Proprietor: **Syngenta Limited**
**Guildford**
**Surrey GU2 7YH (GB)**

(72) Inventors:
- **DOE, John, Ernest**
  **Cheshire SK10 4TJ (GB)**
- **STURGESS, Nicholas, Crispinian**
  **Cheshire SK10 4TJ (GB)**
- **TRAVIS, Kim, Zachary**
  **Cheshire SK10 4TJ (GB)**

(74) Representative: **Marshall, Cameron John et al**
**Carpmaels & Ransford**
**One Southampton Row**
**London**
**WC1B 5HA (GB)**

(56) References cited:
**WO-A-2004/005321**
- **LOCK E A ET AL: "From toxicological problem to therapeutic use: The discovery of the mode of action of 2-(2-nitro-4-trifluoromethylbenzoyl)-1,3-c yclohexan edione (NTBC), its toxicology and development as a drug" JOURNAL OF INHERITED METABOLIC DISEASE, vol. 21, no. 5, August 1998 (1998-08), pages 498-506, XP002376408 ISSN: 0141-8955**
- **ANIKSTER Y ET AL: "NTBC and alkaptonuria." AMERICAN JOURNAL OF HUMAN GENETICS. SEP 1998, vol. 63, no. 3, September 1998 (1998-09), pages 920-921, XP002376409 ISSN: 0002-9297**
- **YANG D-Y: "4-Hydroxyphenylpyruvate Dioxygenase as a Drug Discovery Target" DRUG NEWS AND PERSPECTIVES 2003 SPAIN, vol. 16, no. 8, 2003, pages 493-496, XP001246754 ISSN: 0214-0934**
- **LOCK EDWARD A ET AL: "Tissue distribution of 2-(2-nitro-4-trifluoromethylbenzoyl)cycloh exan e-1,3-dione (NTBC): Effect on enzymes involved in tyrosine catabolism and relevance to ocular toxicity in the rat" TOXICOLOGY AND APPLIED PHARMACOLOGY, vol. 141, no. 2, 1996, pages 439-447, XP002376410 ISSN: 0041-008X**
- **LIN S-W ET AL: "Discovery of a potent, non-triketone type inhibitor of 4-hydroxyphenylpyruvate dioxygenase" BIOORGANIC & MEDICINAL CHEMISTRY LETTERS, OXFORD, GB, vol. 10, no. 11, June 2000 (2000-06), pages 1297-1298, XP004200579 ISSN: 0960-894X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- **GROWDON J H ET AL: "EFFECTS OF ORAL L TYROSINE ADMINISTRATION ON CEREBRO SPINAL FLUID TYROSINE AND HOMO VANILLIC-ACID LEVELS IN PATIENTS WITH PARKINSONS DISEASE" LIFE SCIENCES, vol. 30, no. 10, 1982, pages 827-832, XP001056686 ISSN: 0024-3205**
- **LEMOINE P ET AL: "LA L-TYROSINE: TRAITEMENT AU LONG COURS DE LA MALADIE DE PARKINSON//L-TYROSINE: A LONG TERM TREATMENT OF PARKINSONS'S DISEASE", COMPTES RENDUS - BIOLOGIES, ELSEVIER, PARIS, FR, vol. 309, no. 2, SERIE III, 1 June 1989 (1989-06-01), pages 43-47, XP009064980, ISSN: 1631-0691**
- **LEMOINE P. ET AL: 'LA L-TYROSINE: TRAITEMENT AU LONG COURS DE LA MALADIE DE PARKINSON//L-TYROSINE: A LONG TERM TREATMENT OF PARKINSONS'S DISEASE' COMPTES RENDUS - BIOLOGIES, ELSEVIER, PARIS, FR vol. 309, no. 2, SERIE III, 01 June 1989, pages 43 - 47, XP009064980 ISSN: 1631-0691**

## Description

The present invention relates to, *inter alia,* the use of a 4-hydroxyphenylpyruvate dioxygenase (HPPD) inhibitor in the treatment of neurodegenerative disease. More specifically, the invention relates to the use of a HPPD inhibitor in an amount which is effective to treat Parkinson's disease. In a particular embodiment the HPPD inhibitor is 2-(2-Nitro-4-Trifluoromethylbenzoyl)-1,3-Cyclohexanedione (compound 2).

Neurodegenerative diseases affect millions of people worldwide. In particular, Parkinson's disease is increasing in prevalence due to increasing lifespan. The disease is not very well understood, though a key aspect is oxidative damage, resulting in the loss of dopaminergic neurones in the *substantia nigra* region of the brain and consequential reductions in striatal dopamine. Once striatal dopamine levels have been depleted by approximately 80%, symptoms of Parkinson's become apparent. Such symptoms increase in severity as more neurones are lost. There are numerous publications which provide an overview of the disease. More recent publications provide an overview of treatments for patients suffering from Parkinson's disease.

Virtually all symptomatic treatment of the disease involves increasing the brain supply of dopamine or by systemic administration of dopamine agonists. Dopamine itself cannot cross the blood-brain barrier (BBB), and the dominant drug used is levodopa, the immediate precursor for dopamine, which can readily cross the BBB. Levodopa treatment has to be supplemented with other drugs (e.g. carbidopa) that inhibit the metabolism of levodopa in other parts of the body - this reduces adverse side-effects and increases and extends the levodopa concentration in plasma. Slow-release formulations of levodopa are also used, but the kinetics of levodopa remain far from optimal. After a few years of levodopa therapy, during which time more neurones have been lost in the patient, the effectiveness of levodopa is reduced (referred to clinically as "wearing off') and the therapeutic margin reduces or disappears. Patients experience Parkinsonian symptoms before their next dose is due ("off periods"), yet the dose cannot be increased without causing side-effects, primarily dyskinesia. These side-effects are to a significant extent believed to be the result of the fast kinetics of levodopa, and the pulsatile dopaminergic stimulation that this causes. Dopamine receptor agonists are available in the art and more are being developed, but their potency is limited. Such agonists seem to be of use, primarily, in the early stages of treatment or as adjuncts to levodopa treatment. Other adjunct therapies to levodopa treatment are also used and more are being developed.

More recent research into treatments is aimed at identifying ways to detect the disease before it becomes symptomatic, and to treat it with "neuroprotectants". If completely effective, these would prevent further loss of neurones and so halt disease progression. However pre-symptomatic detection is proving to be extremely difficult. For patients who already have symptoms, any treatment with neuroprotectants would supplement rather than replace drugs for treating the symptoms. Also, at present, neuroprotectants so far only result in, at best, a modest delay in disease progression, rather than halting the disease.

It would therefore be desirable to provide a pharmaceutical which would produce a constant elevation of brain dopamine levels in Parkinson's disease patients. This concept is known as continuous dopaminergic stimulation. Then, treatment would be more effective than systemic levodopa or dopamine agonists and such treatment could maintain its effectiveness for longer, with fewer side-effects.

The present invention therefore seeks to provide, *inter alia,* a pharmaceutical for use in the treatment of Parkinson's disease which pharmaceutical overcomes and/or ameliorates the problems mentioned above.

Accordingly the present invention provides, amongst other things, compositions for their use to inhibit 4-hydroxyphenylpyruvate dioxygenase in animals such that an increase in levodopa and/or dopamine synthesis is achieved.

According to the present invention there is provided the use of 2-(2-Nitro-4-Trifluoromethylbenzoyl)-1,3-Cyclohexanedione (compound 2), or a pharmaceutically acceptable salt thereof, in the manufacture of a medicament for use in the treatment of a neurodegenerative disease.

The present invention further provides the use as described above wherein said disease is Parkinson's disease.

It will be appreciated that 2-(2-Nitro-4-Trifluoromethylbenzoyl)-1,3-Cyclohexanedione may exist in one or more tautomeric forms, one of which is shown in formula (II) (i.e. compound 2) : and which forms are readily inter-convertible by keto-enol tautomerism.

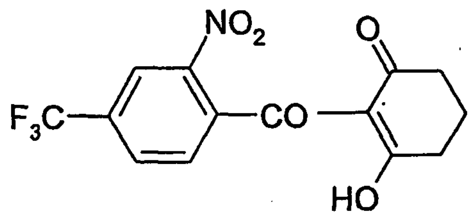

(II)

(Compound 2)

It is to be understood that the invention includes the use of 2-(2-Nitro-4-Trifluoromethylbenzoyl)-1,3-Cyclohexanedione in any of such tautomeric forms or as a mixture thereof.

2-(2-Nitro-4-Trifluoromethylbenzoyl)-1,3-Cyclohexanedione is acidic and readily forms salts with a wide variety of bases.

Particularly suitable salts of 2-(2-Nitro-4-Trifluoromethylbenzoyl)-1,3-Cyclohexanedione suitable for use as active ingredients in pharmaceutical compositions according to the invention include, for example, pharmaceutically acceptable base-addition salts, for example, alkali metal (such as potassium or sodium), alkaline earth metal (such as calcium or magnesium) and ammonium salts, and salts with organic bases giving physiologically acceptable cations (such as salts with methylamine, dimethylamine, trimethylamine, piperidine and morpholine).

2-(2-Nitro-4-Trifluoromethylbenzoyl)-1,3-Cyclohexanedione may be obtained by conventional procedures of organic chemistry already known for the production of structurally analogous materials.

Thus, for example, 2-(2-Nitro-4-Trifluoromethylbenzoyl)-1,3-Cyclohexanedione may be conveniently obtained by reaction of 2-nitro-4-trifluoromethylbenzoyl chloride with cyclohexane-1,3-dione in the presence of acetone cyanhydrin and a suitable base such as triethylamine.

The starting 2-nitro-4-trifluoromethylbenzoyl chloride may itself be obtained from the corresponding benzoic acid, for example by reaction with thionyl chloride or oxalyl chloride as is described in Reagents for Organic Synthesis, (J Wiley and Sons, 1967; editors: Fieser L. F. and Fieser M.; Vol 1, pp. 767-769) and is generally used without special purification.

Similarly, 2-nitro-4-triflurorometh ylbenzoic acid may be obtained, for example, as described by Haupstein et al. in J. Amer. Chem. Soc., 1954, 76, 1051, or by one of the general methods well known to the skilled person.

The present invention still further provides the use as described above wherein the medicament comprises compound 2 or a pharmaceutically acceptable salt thereof and a further compound which is also capable of inhibiting 4-hydroxyphenylpyruvate dioxygenase (HPPD) in an animal.

The present invention still further provides the use as described above wherein said medicament comprises a dopamine agonist.

The present invention still further provides the use as described above wherein said medicament comprises levodopa and a decarboxylase inhibitor.

In a further aspect of the invention there is provided a kit comprising a pharmaceutically effective amount of compound 2 or a pharmaceutically acceptable salt thereof and a pharmaceutically effective amount of a dopamine agonist and a means for the delivery thereof to an animal.

In a still further aspect of the invention there is provided a kit comprising a pharmaceutically effective amount of compound 2 or a pharmaceutically acceptable salt thereof and a pharmaceutically effective amount of levodopa and a means for the delivery thereof to an animal.

In a still further aspect of the invention there is provided a kit comprising a pharmaceutically effective amount of compound 2 or a pharmaceutically acceptable salt thereof and a pharmaceutically effective amount of levodopa and a decarboxylase inhibitor and a means for the delivery thereof to an animal.

In a still further aspect of the invention there is provided a kit comprising a pharmaceutically effective amount of compound 2 or a pharmaceutically acceptable salt thereof and a pharmaceutically effective amount of a catechol-O-methyl transferase inhibitor and a means for the delivery thereof to an animal.

In a still further aspect of the invention there is provided a kit comprising a pharmaceutically effective amount of compound 2 or a pharmaceutically acceptable salt thereof and a pharmaceutically effective amount of a monoamine oxidase inhibitor and a means for the delivery thereof to an animal.

In a still further aspect of the invention there is provided a kit comprising a pharmaceutically effective amount of compound 2 or a pharmaceutically acceptable salt thereof and a pharmaceutically effective amount of a further

compound which is also capable of inhibiting HPPD in an animal and a means for the delivery thereof to an animal.

In a still further aspect of the invention there is provided a pharmaceutical composition comprising as an active ingredient compound 2 or a pharmaceutically acceptable salt thereof and a pharmaceutically effective amount of a further compound which is also capable of inhibiting HPPD in an animal optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of a dopamine agonist optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of levodopa optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of levodopa and a decarboxylase inhibitor optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of a catechol-O-methyl transferase inhibitor optionally together with a pharmaceutically acceptable diluent or carrier.

In a still further aspect of the invention there is provided a pharmaceutical composition comprising a pharmaceutically effective amount of-compound 2 or a pharmaceutically acceptable salt thereof and a pharmaceutically effective amount of a monoamine oxidase inhibitor optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of a decarboxylase inhibitor optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of a neuroprotectant optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of an adenosine (A2a) receptor antagonist optionally together with a pharmaceutically acceptable diluent or carrier.

In a still further aspect of the invention there is provided a pharmaceutical composition comprising a pharmaceutically effective amount of compound 2 or a pharmaceutically acceptable salt thereof and a pharmaceutically effective amount of istradefylline optionally together with a pharmaceutically acceptable diluent or carrier.

The invention still further provides a pharmaceutical composition as described above which is in a form suitable for oral or parenteral administration.

In a particular embodiment, said pharmaceutical composition is in palatable form suitable for oral administration selected from the group consisting of: tablets; lozenges; hard capsules; aqueous suspensions; oily suspensions; emulsions; dispersible powders; dispersible granules; syrups and elixirs.

In a further embodiment said pharmaceutical composition is intended for oral use and is in the form of hard or soft gelatin capsules.

In a still further embodiment said pharmaceutical composition is in a form suitable for parenteral administration.

In a further aspect of the invention there is provided the use of a compound capable of inhibiting 4-hydroxyphenylpyruvate dioxygenase (HPPD) in an animal in the manufacture of a medicament for use in the treatment and/or prevention of a neurodegenerative disease.

HPPD inhibitors that are applicable to the present invention include compounds of formula I (the term formula I may be interchanged with compound 1):

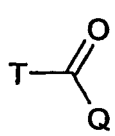 (I) (Compound 1)

wherein;

T is $T_1$

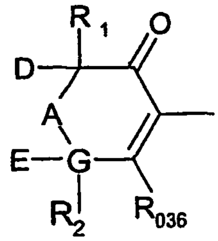

(T1),

Wherein:

G is C or N wherein when G is N then only one of E and $R_2$ are present;
D is hydrogen or $R_3$;
E is hydrogen or $R_4$; or
D and E together are $C_2$-$C_3$alkylene which can be mono- or poly-substituted by $R_6$;
A is $C_1$-$C_2$alkylene which can be mono- or poly-substituted by $R_5$; or A may additionally be carbonyl, oxygen or -N-$R_7$- when D and E are other than $C_2$-$C_3$alkylene;
$R_1$, $R_2$, $R_3$, $R_4$, $R_5$ and $R_6$ are each independently of the others hydrogen, $C_1$-$C_4$alkyl, phenyl, $C_1$-$C_4$alkoxy, halogen, hydroxy, cyano, hydroxycarbonyl or $C_1$-$C_4$alkoxycarbonyl;
or $R_2$ and $R_4$ together form a $C_2$-$C_4$alkylene chain which can be interrupted by oxygen and/or carbonyl and/or sulfur, with the proviso that the oxygen and sulfur atoms are separated by at least one methylene group;
$R_7$ is $C_1$-$C_4$alkyl, alkoxycarbonyl or $C_1$-C4alkylcarbonyl;
$R_{036}$ is hydroxy, $O^-M^+$, wherein $M^+$ is an alkali metal cation or ammonium cation, halogen, $C_1$-$C_{12}$alkylsulfonyloxy, amino, $C_1$-$C_4$alkylthio, $C_1$-$C_{12}$alkylsulfinyl, $C_1$-$C_{12}$alkylsulfonyl, $C_1$-$C_{12}$haloalkylthio, $C_1$-$C_{12}$haloalkylsulfinyl, $C_1$-$C_{12}$haloalkylsulfonyl, $C_1$-$C_6$alkoxy-$C_1$-$C_6$alkylthio, $C_1$-$C_6$alkoxy-$C_1$-$C_6$alkylsulfinyl, $C_1$-$C_6$alkoxy-$C_1$-$C_6$alkylsulfonyl, $C_3$-$C_{12}$alkenylthio, $C_3$-$C_{12}$alkenylsulfinyl, $C_3$-$C_{12}$alkenylsulfonyl, $C_3$-$C_{12}$alkynylthio, $C_3$-$C_{12}$alkynylsulfinyl, $C_3$-$C_{12}$alkynylsulfonyl, $C_1$-$C_4$alkoxycarbonyl-$C_1$-$C_4$alkylthio, $C_1$-$C_4$alkoxycarbonyl-$C_1$-$C_4$alkylsulfinyl, $C_1$-$C_4$alkoxycarbonyl-$C_1$-$C_4$alkylsulfonyl, $(C_1$-$C_4alkoxy)_2P(O)O$, $C_1$-$C_4$alkyl-($C_1$-$C_4$alkoxy)P(O)O, H($C_1$-$C_4$alkoxy)P(O)O, $R_{037}R_{038}N$, $R_{039}R_{040}NNH$, $R_{041}R_{042}NC(O)O$-, $R_{043}R_{044}NC(O)NH$-, $C_1$-$C_{18}$alkylcarbonyloxy, $C_2$-$C_{18}$alkenylcarbonyloxy, $C_2$-$C_{18}$alkynylcarbonyloxy, $C_3$-$C_6$cycloalkylcarbonyloxy, $C_1$-$C_{12}$alkoxycarbonyloxy, $C_1$-$C_{12}$alkylthiocarbonyloxy or $C_1$-$C_{12}$alkylthiocarbamoyl, wherein the alkyl, alkenyl and alkynyl groups can be substituted by halogen, $C_1$-$C_6$alkoxy, $C_1$-$C_6$alkylthio, $C_1$-$C_6$alkylsulfinyl, $C_1$-$C_6$alkylsulfonyl or by cyano; or
$R_{036}$ is phenoxy, phenylthio, phenylsulfinyl, phenylsulfonyl, phenylsulfonylamino, phenylsulfonyloxy, benzoyloxy or benzoyl-$C_1$-$C_6$alkoxy, wherein the phenyl groups can in turn be substituted one or more times by halogen, nitro, cyano, $C_1$-$C_4$alkyl, $C_1$-$C_4$haloalkyl, $C_1$-$C_4$alkoxy and/or $C_1$-$C_4$haloalkoxy,
or $R_{036}$ is a group $Het_{07}$-thio, $Het_{08}$-sulfinyl, $Het_{09}$-sulfonyl, $Het_{010}$-(CO)O or $Het_{011}$-N($R_{047}$); wherein
$Het_{07}$, $Het_{08}$, $Het_{09}$, $Het_{010}$ and $Het_{011}$ are each independently of the others a five- to ten-membered monocyclic or annellated bicyclic ring system which may be aromatic or partially saturated and may contain from 1 to 4 hetero atoms elected from nitrogen, oxygen and sulfur, and each ring system may contain not more than two oxygen atoms and not more than two sulfur atoms, and the ring system itself can be substituted by $C_1$-$C_6$alkyl, $C_1$-$C_6$haloalkyl, $C_1$-$C_6$alkoxy, $C_1$-$C_6$haloalkoxy, $C_1$-$C_6$alkylthio, $C_1$-$C_6$alkylsulfinyl, $C_1$-$C_6$alkylsulfonyl, di($C_1$-$C_4$alkyl)aminosulfonyl, di($C_1$-$C_4$alkyl)amino, halogen, cyano, nitro or by phenyl, and the substituents on the nitrogen atom in the heterocyclic ring are other than halogen;
$R_{037}$, $R_{038}$, $R_{039}$, $R_{040}$, $R_{041}$, $R_{042}$, $R_{043}$, $R_{044}$ and $R_{047}$ are each independently of the others hydrogen or $C_1$-$C_6$alkyl; or
$R_{037}$ and $R_{038}$ together or $R_{039}$ and $R_{040}$ together or $R_{041}$ and $R_{042}$ together or $R_{043}$ and $R_{044}$ together are pyrrolidino, piperidino, morpholino or thiomorpholino, which can be mono-or poly-substituted by methyl groups;
or T is $T_2$

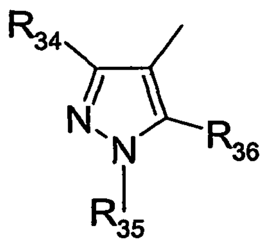

(T$_2$);

wherein

$R_{34}$ is hydrogen, $C_1$-$C_4$alkyl, $C_1$-$C_4$haloalkyl, $C_3$-$C_6$cycloalkyl, $C_2$-$C_4$alkenyl, $C_2$-$C_4$alkynyl or benzyl, it being possible for the phenyl group to be substituted one or more times by $C_1$-$C_6$alkyl, $C_1$-$C_6$haloalkyl, $C_1$-$C_6$alkoxy, $C_1$-$C_6$haloalkoxy, halogen, cyano, hydroxy and/or nitro;

$R_{35}$ is hydrogen, $C_1$-$C_4$alkyl, $C_1$-$C_4$haloalkyl, $C_3$-$C_6$cycloalkyl, $C_3$-$C_4$alkenyl, $C_3$-$C_4$alkynyl or benzyl, it being possible for the phenyl group to be substituted one or more times by $C_1$-$C_6$alkyl, $C_1$-$C_6$haloalkyl, $C_1$-$C_6$allcoxy, $C_1$-$C_6$haloalkoxy, halogen, cyano, hydroxy and/or nitro;

$R_{36}$ is hydroxy, $O^-M^+$, wherein $M^+$ is an alkali metal cation or ammonium cation, halogen, $C_1$-$C_{12}$alkylsulfonyloxy, amino, $C_1$-$C_4$alkylthio, $C_1$-$C_{12}$alkylsulfinyl, $C_1$-$C_{12}$alkylsulfonyl, $C_1$-$C_{12}$haloalkylthio, $C_1$-$C_{12}$haloalkylsulfinyl, $C_1$-$C_{12}$haloakylsulfonyl, $C_1$-$C_6$alkoxy-$C_1$-$C_6$alkylthio, $C_1$-$C_6$alkoxy-$C_1$-$C_6$alkylsulfinyl, $C_1$-$C_6$alkoxy-$C_1$-$C_6$alkylsulfonyl, $C_3$-$C_{12}$alkenylthio, $C_3$-$C_{12}$alkenylsulfinyl, $C_3$-$C_{12}$alkenylsulfonyl, $C_3$-$C_{12}$alkynylthio, $C_3$-$C_{12}$alkynylsulfinyl, $C_3$-$C_{12}$alkynylsulfonyl, $C_1$-$C_4$alkoxycarbonyl-$C_1$-$C_4$alkylthio, $C_1$-$C_4$alkoxycarbonyl-$C_1$-$C_4$alkylsulfinyl, $C_1$-$C_4$alkoxycarbonyl-$C_1$-$C_4$alkylsulfonyl, $(C_1$-$C_4$alkoxy$)_2$P(O)O, $C_1$-$C_4$alkyl-($C_1$-$C_4$alkoxy)P(O)O, H(C)-$C_4$alkoxy)P(O)O, $R_{37}R_{38}$N, $R_{39}R_{40}$NNH, $R_{41}R_{42}$NC(O)O-, $R_{43}R_{44}$NC(O)NH-, $C_1$-$C_{18}$alkylcarbonyloxy, $C_2$-$C_{18}$alkenylcarbonyloxy, $C_2$-$C_{18}$alkynylcarbonyloxy, $C_3$-$C_6$-cycloalkylcarbonyloxy, $C_1$-$C_{12}$alkoxycarbonyloxy, $C_1$-$C_{12}$alkylthiocarbonyloxy or $C_1$-$C_{12}$alkylthiocarbamoyl, wherein the alkyl, alkenyl and alkynyl groups can be substituted by halogen, $C_1$-$C_6$alkoxy, $C_1$-$C_6$alkylthio, $C_1$-$C_6$alkylsulfinyl, $C_1$-$C_6$alkylsulfonyl or by cyano; or

$R_{36}$ is phenoxy, phenylthio, phenylsulfinyl, phenylsulfonyl, phenylsulfonylamino, phenylsulfonyloxy, benzoyloxy or benzoyl-$C_1$-$C_6$alkoxy, it being possible for the phenyl groups in turn to be substituted one or more times by halogen, nitro, cyano, $C_1$-$C_4$alkyl, $C_1$-$C_4$haloalkyl, $C_1$-$C_4$alkoxy and/or $C_1$-$C_4$haloalkoxy,

or $R_{36}$ is a group Het$_7$-thio, Het$_8$-sulfinyl, Het$_9$-sulfonyl, Het$_{10}$-(CO)O or Het$_{11}$-N($R_{47}$); wherein

Het$_7$, Het$_8$, Het$_9$, Het$_{10}$ and Het$_{11}$ are each independently of the others a five- to ten-membered monocyclic or annellated bicyclic ring system which may be aromatic or partially saturated and may contain from 1 to 4 hetero atoms selected from nitrogen, oxygen and sulfur, and each ring system may contain not more than two oxygen atoms and not more than two sulfur atoms, and the ring system itself can be substituted by $C_1$-$C_6$alkyl, $C_1$-$C_6$haloalkyl, $C_1$-$C_6$alkoxy, $C_1$-$C_6$haloalkoxy, $C_1$-$C_6$alkylthio, $C_1$-$C_6$alkylsulfinyl, $C_1$-$C_6$alkylsulfonyl, di($C_1$-$C_4$alkyl)aminosulfonyl, di($C_1$-$C_4$alkyl)amino, halogen, cyano, nitro or by phenyl, and the substituents on the nitrogen atom in the heterocyclic ring are other than halogen;

$R_{37}$, $R_{38}$, $R_{39}$, $R_{40}$, $R_{41}$, $R_{42}$, $R_{43}$, $R_{44}$ and $R_{47}$ are each independently of the others hydrogen or $C_1$-$C_6$alkyl; or

$R_{37}$ and $R_{38}$ together or $R_{39}$ and $R_{40}$ together or $R_{41}$ and $R_{42}$ together or $R_{43}$ and $R_{44}$ together are pyrrolidino, piperidino, morpholino or thiomorpholino, which can be mono-or poly-substituted by methyl groups;

or T is T$_3$

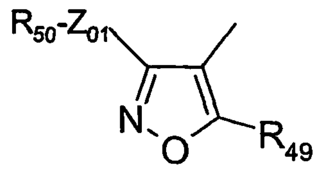

(T$_3$);

wherein

$R_{49}$ is $C_1$-$C_4$alkyl, $C_1$-$C_4$haloalkyl, $C_3$-$C_6$cycloalkyl or halo-substituted $C_3$-$C_6$cycloalkyl; $Z_{01}$ is a chemical bond, S, SO or $SO_2$; or $-CO_2$-

$R_{50}$ is hydrogen or $C_1$-$C_3$alkylene which can be substituted by the following substituents: halogen, hydroxy, $C_1$-$C_6$alkoxy, $C_2$-$C_6$alkenyl, $C_2$-$C_6$alkynyl, $C_3$-$C_6$cycloalkyl, $C_1$-$C_6$alkoxy-$C_1$-$C_6$alkoxy, $C_1$-$C_6$alkoxy-$C_1$-$C_6$alkoxy-$C_1$-$C_6$alkoxy, (3-oxetanyl)-oxy, $C_1$-$C_6$alkyl-substituted (3-oxetanyl)-oxy, benzylthio, benzylsulfinyl, benzylsulfonyl,

phenyl, phenoxy, phenylthio, phenylsulfinyl or phenylsulfonyl, it being possible for the phenyl-and benzyl-containing groups in turn to be substituted by one or more $C_1$-$C_6$alkyl, $C_1$-$C_6$haloalkyl, $C_1$-$C_6$alkoxy, $C_1$-$C_6$haloalkoxy, halogen, cyano, hydroxy and/or nitro groups;

or $R_{50}$ is phenyl, it being possible for the phenyl-containing group in turn to be substituted by one or more $C_1$-$C_6$alkyl, $C_1$-$C_6$haloalkyl, $C_1$-$C_6$alkoxy, $C_1$-$C_6$haloalkoxy, halogen, cyano, hydroxy and/or nitro groups,

or $R_{50}$ is $C_3$-$C_6$cycloalkyl, $C_1$-$C_6$alkoxy- or $C_1$-$C_6$alkyl-substituted $C_3$-$C_6$cycloalkyl, 3-oxetanyl or $C_1$-$C_6$alkyl-substituted 3-oxetanyl;

or T is $T_4$

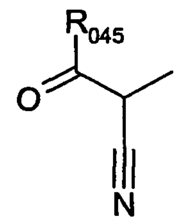

($T_4$);

wherein

$R_{045}$ is $C_1$-$C_6$alkyl, $C_1$-$C_6$haloalkyl, $C_3$-$C_6$cycloalkyl or halo-substituted $C_3$-$C_6$cycloalkyl; and their pharmaceutically acceptable salts, isomers and enantiomers.

The compounds of formula I also include the salts which such compounds are able to form with amines, alkali metal and alkaline earth metal bases or quaternary ammonium bases. Among the alkali metal and alkaline earth metal hydroxides as salt formers, special mention should be made of the hydroxides of lithium, sodium, potassium, magnesium and calcium, but especially the hydroxides of sodium and potassium.

Examples of amines suitable for ammonium salt formation include ammonia as well as primary, secondary and tertiary $C_1$-$C_{18}$alkylamines, $C_1$-$C_4$hydroxyalkylamines and $C_2$-$C_4$alkoxyalkylamines, for example methylamine, ethylamine, n-propylamine, isopropylamine, the four butylamine isomers, n-amylamine, isoamylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, diisopropylamine, din-butylamine, di-n-amylamine, diisoamylamine, dihexylamine, diheptylamine, dioctylamine, ethanolamine, n-propanolamine, isopropanolamine, N,N-diethanolamine, N-ethylpropanolamine, N-butylethanolamine, allylamine, n-butenyl-2-amine, n-pentenyl-2-amine, 2,3-dimethylbutenyl-2-amine, dibutenyl-2-amine, n-hexenyl-2-amine, propylenediamine, trimethylamine, triethylamine, tri-n-propylamine, triisopropylamine, tri-n-butylamine, triisobutylamine, tri-sec-butylamine, tri-n-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, o-, m- and p-toluidines, phenylenediamines, benzidines, naphthylamines and o-, m- and p-chloroanilines; but especially triethylamine, isopropylamine and diisopropylamine.

Because the compounds of formula I wherein T is $T_1$ are preferably in enolised forms or in the form of salts, formula I also includes the enolised forms of formulae Ia, Ib, Ic and Id wherein M is hydrogen or a metal ion or an ammonium ion.

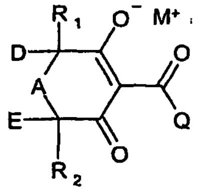

(Ia)

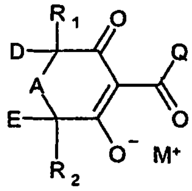

(Ib)

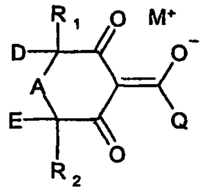

(Ic)

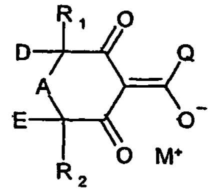

(Id)

Since compounds of formula I may also contain asymmetric carbon atoms, for example in the case of the

carbon atom carrying $R_1$, D and A, all stereoisomeric forms are also included.

The organic substituent Q may be an inert substituent of any desired structure, provided that the compounds of formula I retain their action as HPPD inhibitors in animals. Such tests of these compounds may be carried out in accordance with the experimental methods described herein.

Q is preferably a mono- or poly-substituted phenyl, pyridyl or heteroaryl group, especially 2-benzoyl, 2-isonicotinoyl and 2-nicotinoyl derivatives, the substitution pattern of those groups being freely selectable provided that the compounds of formula I retain their action as HPPD inhibitors in animals.

In a particular embodiment said HPPD inhibitors are compounds of formula I wherein
Q is $Q_1$

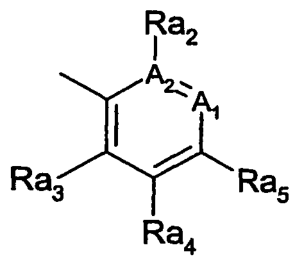
($Q_1$),

wherein
$A_1$ or $A_2$ are independently selected from methine, $C(Ra_1)$ or N(O)p; (wherein preferably at least one of $A_1$ or $A_2$ is methine p is 0 or 1;
$Ra_1$ is hydrogen, $C_1$-$C_6$alkyl, hydroxy, $C_1$-$C_6$alkoxy, $C_1$-$C_6$haloallcoxy, $C_3$-$C_6$alkenyloxy, $C_3$-$C_6$haloalkenyloxy, $C_3$-$C_6$alkynyloxy, $C_1$-$C_4$alkylcarbonyloxy, $C_1$-$C_4$alkylsulfonyloxy, tosyloxy, $C_1$-$C_4$alkylthio, $C_1$-$C_4$alkylsulfinyl, $C_1$-$C_4$alkylsulfonyl, $C_1$-$C_4$alkylamino, di-$C_1$-$C_4$alkylamino, $C_1$-$C_4$alkoxycarbonyl, $C_1$-$C_4$haloalkyl, formyl, cyano, halogen, phenyl or phenoxy; it being possible for phenyl in turn to be substituted by $C_1$-$C_3$alkyl, $C_1$-$C_3$haloalkyl, $C_1$-$C_3$alkoxy, $C_1$-$C_3$haloalkoxy, halogen, cyano or by nitro; or $Ra_1$ is a three- to ten-membered monocyclic ring system or, together with $Ra_2$ or Ras, annellated mono- or bi-cyclic ring system which may be interrupted by oxygen, sulfur, SO, $SO_2$, $NRa_6$, carbonyl and/or by $=NORa_7$, the ring system, unless it is annellated, being bonded to the carbon atom of the substituent $A_1$ directly or by way of a $C_1$-$C_4$alkylene, -CH=CH-, -C≡C-, $-CH_2O-$, $-CH_2N(C_1$-$C_4$alkyl)-, $-CH_2S-$, $-CH_2SO-$ or - $CH_2SO_2$-group, and the ring system may contain not more than two oxygen atoms and not more than two sulfur atoms, and the ring system can itself be mono-, di- or tri-substituted by $C_1$-$C_6$alkyl, $C_1$-$C_6$haloalkyl, $C_2$-$C_6$alkenyl, $C_2$-$C_6$haloalkenyl, $C_2$-$C_6$alkynyl, $C_2$-$C_6$haloalkynyl, $C_1$-$C_6$alkoxy, $C_1$-$C_6$haloalkoxy, $C_3$-$C_6$akenyloxy, $C_3$-$C_6$alkynyloxy, $C_1$-$C_6$alkylthio, $C_1$-$C_6$haloalkylthio, $C_3$-$C_6$alkenylthio, $C_3$-$C_6$halo-alkenylthio, $C_3$-$C_6$alkynylthio, $C_1$-$C_4$alkoxy-$C_1$-$C_2$alkylthio, $C_1$-$C_4$alkylcarbonyl-$C_1$-$C_2$alkylthio, $C_1$-$C_4$alkoxycarbonyl-$C_1$-$C_2$alkylthio, cyano-$C_1$-$C_4$alkylthio, $C_1$-$C_6$alkylsulfinyl, $C_1$-$C_6$haloalkylsulfinyl, $C_1$-$C_6$alkylsulfonyl, $C_1$-$C_6$haloalkylsulfonyl, aminosulfonyl, $C_1$-$C_2$alkylaminosulfonyl, di ($C_1$-$C_2$alkyl)aminosulfonyl, di($C_1$-$C_4$alkyl)amino, halogen, cyano, nitro, phenyl and/or benzylthio, it being possible for phenyl and benzylthio in turn to be substituted on the phenyl ring by $C_1$-$C_3$alkyl, $C_1$-$C_3$haloalkyl, $C_1$-$C_3$alkoxy, $C_1$-$C_3$haloalkoxy, halogen, cyano or by nitro, and substituents on the nitrogen atom in the heterocyclic ring are other than halogen;
or $Ra_1$ is the group $-X_5$-$X_7$ or the group $-X_6$-$X_5$-$X_7$; wherein
$X_6$ is a $C_1$-$C_6$alkylene, $C_3$-$C_6$alkenylene or $C_3$-$C_6$alkynylene chain which can be mono- or poly-substituted by halogen and/or by $X_8$, the unsaturated bonds of the chain not being bonded directly to the substituent $X_5$;
$X_8$ is hydroxy, $C_1$-$C_6$alkoxy, $C_3$-$C_6$cycloalkyloxy, $C_1$-$C_6$alkoxy-$C_1$-$C_6$alkoxy, $C_1$-$C_6$alkoxy-$C_1$-$C_6$alkoxy-$C_1$-$C_6$alkoxy or $C_1$-$C_2$alkylsulfonyloxy;
$X_5$ is oxygen, -O(CO)-, -(CO)O-, -O(CO)O-, -N($C_1$-$C_4$alkyl)-O-, -O-N($C_1$-$C_4$alkyl)-, thio, sulfinyl, sulfonyl, $-SO_2N$ ($C_1$-$C_4$alkyl)-, -N($C_1$-$C_4$alkoxy)$SO_2$-, -N($C_1$-$C_4$alkyl)$SO_2$-, -N($C_1$-$C_2$alkoxy-$C_1$-$C_2$alkyl)$SO_2$- or -N($C_1$-$C_4$alkyl)-;
$Ra_6$ is hydrogen, $C_1$-$C_4$alkyl, $C_1$-$C_4$alkylthio-$C_1$-$C_4$carbonyl, $C_1$-$C_4$alkylsulfinyl-$C_1$-$C_4$carbonyl, $C_1$-$C_4$alkylsulfonyl-$C_1$-$C_4$carbonyl, $C_1$-$C_4$alkoxycarbonyl, $C_1$-$C_4$alkylcarbonyl, phenylcarbonyl or phenyl, it being possible for the phenyl groups in turn to be substituted by $C_1$-$C_4$alkyl, $C_1$-$C_4$haloalkyl, $C_1$-$C_4$alkoxy, $C_1$-$C_4$haloalkoxy, $C_1$-$C_4$alkylcarbonyl, $C_1$-$C_4$alkoxycarbonyl, $C_1$-$C_4$alkylamino, di-$C_1$-$C_4$alkylamino, $C_1$-$C_4$alkyl-S-, $C_1$-$C_4$alkyl-SO-, $C_1$-$C_4$alkyl-$SO_2$, $C_1$-$C_4$alkyl-$S(O)_2O$, $C_1$-$C_4$haloalkyl-S-, $C_1$-$C_4$haloalkyl-SO, $C_1$-$C_4$haloalkyl-$SO_2$, $C_1$-$C_4$haloalkyl-$S(O)_2O$, $C_1$-$C_4$alkyl-S $(O)_2NH$, $C_1$-$C_4$alkyl-$S(O)_2N$($C_1$-$C_4$alkyl), halogen, nitro or by cyano;
$Ra_7$ is $C_1$-$C_4$alkyl;
$Ra_2$ is hydrogen, $C_1$-$C_6$alkyl, $C_1$-$C_6$haloalkyl, $C_2$-$C_6$alkenyl, $C_2$-$C_6$haloalkenyl, vinyl substituted by $C_1$-$C_2$alkoxycarbonyl or by phenyl, $C_2$-$C_6$alkynyl, $C_2$-$C_6$haloalkynyl, ethynyl substituted by trimethylsilyl, hydroxy, $C_1$-$C_2$alkoxy,

$C_1$-$C_2$alkoxycarbonyl or by phenyl, $C_3$-$C_6$allenyl, $C_3$-$C_6$cycloalkyl, halo-substituted $C_3$-$C_6$cycloalkyl, $C_1$-$C_6$alkoxy, $C_3$-$C_6$alkenyloxy, $C_3$-$C_6$alkynyloxy, $C_1$-$C_6$haloalkoxy, $C_3$-$C_6$haloalkenyloxy, cyano-$C_1$-$C_4$alkoxy, $C_1$-$C_4$alkoxy-$C_1$-$C_4$alkoxy, $C_1$-$C_4$alkylthio-$C_1$-$C_4$alkoxy, $C_1$-$C_4$alkylsulfinyl-$C_1$-$C_4$alkoxy, $C_1$-$C_4$alkylsulfonyl-$C_1$-$C_4$alkoxy, $C_1$-$C_4$alkoxycarbonyl-$C_1$-$C_4$alkoxy; $C_1$-$C_6$alkylthio, $C_1$-$C_6$alkylsulfinyl, $C_1$-$C_6$alkylsulfonyl, $C_1$-$C_6$haloalkylthio, $C_1$-$C_6$haloalkylsulfinyl, $C_1$-$C_6$haloalkylsulfonyl, $C_1$-$C_4$alkoxycarbonyl-$C_1$-$C_4$alkylthio, $C_1$-$C_4$alkoxycarbonyl-$C_1$-$C_4$alkylsulfinyl, $C_1$-$C_4$alkoxycarbonyl-$C_1$-$C_4$alkylsulfonyl, benzyl-S-, benzyl-SO-, benzyl-$SO_2$-, $C_1$-$C_6$alkylamino, di-$C_2$-$C_6$alkylamino, $C_1$-$C_6$alkylamino-sulfonyl, di($C_1$-$C_6$alkylamino)sulfonyl, benzyloxy, benzyl, phenyl, phenoxy, phenylthio, phenylsulfinyl or phenylsulfonyl, it being possible for the phenyl-containing groups in turn to be substituted by $C_1$-$C_3$alkyl, $C_1$-$C_3$haloalkyl, $C_1$-$C_3$alkoxy, $C_1$-$C_3$haloalkoxy, halogen, cyano or by nitro, or $Ra_2$ is OS-$C_1$-$C_4$alkyl, OSO-$C_1$-$C_4$alkyl, $OSO_2$-$C_1$-$C_4$alkyl, OS-$C_1$-$C_4$haloalkyl, OSO-$C_1$-$C_4$haloalkyl, $OSO_2$-$C_1$-$C_4$haloalkyl, N($C_1$-$C_4$alkyl)-S-$C_1$-$C_4$alkyl, N($C_1$-$C_4$alkyl)-SO-$C_1$-$C_4$alkyl, N($C_1$-$C_4$alkyl)-$SO_2$-$C_1$-$C_4$alkyl, cyano, carbamoyl, $C_1$-$C_4$alkoxycarbonyl, formyl, halogen, rhodano, amino, hydroxy-$C_1$-$C_4$alkyl, $C_1$-$C_4$alkoxy-$C_1$-$C_4$alkyl, $C_1$-$C_4$alkyl-S-$C_1$-$C_4$alkyl, $C_1$-$C_4$alkyl-SO-$C_1$-$C_4$alkyl, $C_1$-$C_4$alkyl-$SO_2$-$C_1$-$C_4$alkyl, cyano-$C_1$-$C_4$alkyl, $C_1$-$C_6$alkylcarbonyloxy-$C_1$-$C_4$alkyl, $C_1$-$C_4$alkoxycarbonyl-$C_1$-$C_4$alkyl, $C_1$-$C_4$alkoxycarbonyloxy-$C_1$-$C_4$alkyl, $C_1$-$C_4$rhodano-$C_l$-$C_4$alkyl, benzoyloxy-$C_1$-$C_4$alkyl, $C_2$-$C_6$oxiranyl, $C_1$-$C_4$alkylamino-$C_1$-$C_4$alkyl, di($C_1$-$C_4$alkyl)amino-$C_1$-$C_4$alkyl, $C_1$-$C_{12}$alkylthiocarbonyl-$C_1$-$C_4$alkyl or formyl-$C_1$-$C_4$alkyl, or $Ra_2$ is a five- to ten-membered monocyclic or annellated bicyclic ring system which may be aromatic or partially saturated and may contain from 1 to 4 hetero atoms selected from nitrogen, oxygen and sulfur, the ring system being bonded to the pyridine ring by way of a $C_1$-$C_4$alkylene, -CH=CH-, -C≡C-, -$CH_2$O-, -$CH_2$N($C_1$-$C_4$alkyl)-, -$CH_2$SO- or -$CH_2SO_2$-group, and each ring system may contain not more than two oxygen atoms and not more than two sulfur atoms, and the ring system itself can be mono-, di- or tri-substituted by $C_1$-$C_6$alkyl, $C_1$-$C_6$haloalkyl, $C_3$-$C_6$alkenyl, $C_3$-$C_6$haloalkenyl, $C_3$-$C_6$alkynyl, $C_3$-$C_6$haloalkynyl, $C_1$-$C_6$alkoxy, $C_1$-$C_6$haloalkoxy, $C_3$-$C_6$alkenyloxy, $C_3$-$C_6$alkynyloxy, mercapto, $C_1$-$C_6$alkylthio, $C_1$-$C_6$haloalkylthio, $C_3$-$C_6$alkenylthio, $C_3$-$C_6$haloalkenylthio, $C_3$-$C_6$alkynylthio, $C_2$-$C_5$alkoxyalkylthio, $C_3$-$C_5$acetylalkylthio, $C_3$-$C_6$alkoxycarbonylalkylthio, $C_2$-$C_4$cyanoalkylthio, $C_1$-$C_6$alkylsulfinyl, $C_1$-$C_6$-haloalkylsulfinyl, $C_1$-$C_6$alkylsulfonyl, $C_1$-$C_6$haloalkylsulfonyl, aminosulfonyl, $C_1$-$C_2$alkylaminosulfonyl, di($C_1$-$C_2$alkyl)aminosulfonyl, di($C_1$-$C_4$alkyl)amino, halogen, cyano, nitro, phenyl and/or by benzylthio, it being possible for phenyl and benzylthio in turn to be substituted on the phenyl ring by $C_1$-$C_3$alkyl, $C_1$-$C_3$haloalkyl, $C_1$-$C_3$alkoxy, $C_1$-$C_3$haloalkoxy, halogen, cyano or by nitro, and substituents on the nitrogen atom in the heterocyclic ring are other than halogen;

or $Ra_2$ is the group -$X_1$-$X_3$ or the group -$X_2$-$X_1$-$X_3$; wherein

$X_2$ is a $C_1$-$C_6$alkylene, $C_3$-$C_6$alkenylene or $C_3$-$C_6$alkynylene chain which can be mono- or poly-substituted by halogen or by $X_4$, the unsaturated bonds of the chain not being bonded directly to the substituent $X_1$;

$X_4$ is hydroxy, $C_1$-$C_6$alkoxy, $C_3$-$C_6$cycloalkyloxy, $C_1$-$C_6$alkoxy-$C_1$-$C_6$alkoxy, $C_1$-$C_6$alkoxy-$C_1$-$C_6$alkoxy-$C_1$-$C_6$alkoxy or $C_1$-$C_2$alkylsulfonyloxy;

$X_1$ is oxygen, -O(CO)-, -(CO)O-, -O(CO)O-, -N($C_1$-$C_4$alkyl)-O-, -O-N($C_1$-$C_4$alkyl)-, thio, sulfinyl, sulfonyl, -$SO_2$N($C_1$-$C_4$alkyl)-, -N($C_1$-$C_4$alkyl)$SO_2$-, -N($C_1$-$C_2$alkoxy-$C_1$-$C_2$alkyl)$SO_2$- or -N($C_1$-$C_4$alkyl)-;

$X_3$ and $X_7$ are each independently of the other a $C_1$-$C_8$alkyl, $C_3$-$C_6$alkenyl or $C_3$-$C_6$alkynyl group which is mono- or poly-substituted by the following substituents: halogen, hydroxy, amino, formyl, nitro, cyano, mercapto, carbamoyl, $C_1$-$C_6$alkoxy, $C_1$-$C_6$alkoxycarbonyl, $C_2$-$C_6$alkenyl, $C_2$-$C_6$haloalkenyl, $C_2$-$C_6$alkynyl, $C_2$-$C_6$haloalkynyl, $C_3$-$C_6$cycloalkyl, halo-substituted $C_3$-$C_6$cycloalkyl, $C_3$-$C_6$alkenyloxy, $C_3$-$C_6$alkynyloxy, $C_1$-$C_6$haloalkoxy, $C_3$-$C_6$haloalkenyloxy, cyano-$C_1$-$C_6$alkoxy, $C_1$-$C_6$alkoxy-$C_1$-$C_6$alkoxy, $C_1$-$C_6$alkoxy-$C_1$-$C_6$alkoxy-$C_1$-Csalkoxy, $C_1$-$C_6$alkylthio-$C_1$-$C_6$alkoxy, $C_1$-$C_6$alkylsulfinyl-$C_1$-$C_6$alkoxy, $C_1$-$C_6$alkylsulfonyl-$C_1$-$C_6$alkoxy, $C_1$-$C_6$alkoxycarbonyl-$C_1$-$C_6$alkoxy, $C_1$-$C_6$alkoxycarbonyl, $C_1$-$C_6$alkylcarbonyl, $C_1$-$C_6$alkylthio, $C_1$-$C_6$alkylsulfinyl, $C_1$-$C_6$-alkylsulfonyl, $C_1$-$C_6$haloalkylthio, $C_1$-$C_6$haloalkylsulfinyl, $C_1$-$C_6$haloalkylsulfonyl, oxiranyl, which can in turn be substituted by $C_1$-$C_6$alkyl, (3-oxetanyl)-oxy, which can in turn be substituted by $C_1$-$C_6$alkyl, benzylthio, benzylsulfinyl, benzylsulfonyl, $C_1$-$C_6$alkylamino, di($C_1$-$C_6$alkyl)amino, $C_1$-$C_4$alkyl-S(O)$_2$O, $C_1$-$C_4$alkyl-N($C_1$-$C_4$alkyl)$SO_2$-, rhodano, phenyl, phenoxy, phenylthio, phenylsulfinyl and/or phenylsulfonyl;

it being possible for the phenyl- or benzyl-containing groups in turn to be substituted by one or more $C_1$-$C_6$alkyl, $C_1$-$C_6$haloalkyl, $C_1$-$C_6$alkoxy, $C_1$-$C_6$haloalkoxy, halogen, cyano, hydroxy and/or nitro groups, or

$X_3$ and $X_7$ are each independently of the other phenyl which can be substituted one or more times by $C_1$-$C_6$alkyl, $C_1$-$C_6$haloalkyl, $C_1$-$C_6$alkoxy, $C_1$-$C_6$haloalkoxy, halogen, cyano, hydroxy and/or nitro; or

$X_3$ and $X_7$ are each independently of the other $C_3$-$C_6$cycloalkyl, $C_1$-$C_6$alkoxy- or $C_1$-$C_6$alkyl-substituted $C_3$-$C_6$cycloalkyl, 3-oxetanyl or $C_1$-$C_6$alkyl-substituted 3-oxetanyl;

or $X_3$ and $X_7$ are each independently of the other a five- to ten-membered monocyclic or annellated bicyclic ring system which may be aromatic or saturated or partially saturated and may contain from 1 to 4 hetero atoms selected from nitrogen, oxygen and sulfur, the ring system being bonded to the substituent $X_1$ or $X_5$ directly or by way of a $C_1$-$C_4$alkylene, $C_2$-$C_4$alkenyl-$C_1$-$C_4$alkylene, $C_2$-$C_4$alkynyl-$C_1$-$C_4$alkylene, -N($C_1$-$C_4$alkyl)-$C_1$-$C_4$alkylene, -SO-$C_1$-$C_4$alkylene or -$SO_2$-$C_1$-$C_4$alkylene group, and each ring system may contain not more than two oxygen atoms and not more than two sulfur atoms, and the ring system can itself be mono-, di- or tri-substituted by $C_1$-$C_6$alkyl, $C_1$-$C_6$haloalkyl, $C_2$-$C_6$alkenyl,

$C_2$-$C_6$haloalkenyl, $C_2$-$C_6$alkynyl, $C_2$-$C_6$haloalkynyl, $C_1$-$C_6$alkoxy, hydroxy, $C_1$-$C_6$haloalkoxy, $C_3$-$C_6$alkenyloxy, $C_3$-$C_6$alkynyloxy, mercapto, $C_1$-$C_6$alkylthio, $C_1$-$C_6$haloalkylthio, $C_3$-$C_6$alkenylthio, $C_3$-$C_6$haloalkenylthio, $C_3$-$C_6$alkynylthio, $C_2$-$C_5$alkoxyalkylthio, $C_3$-$C_5$acetylalkylthio, $C_3$-$C_6$alkoxycarbonylalkylthio, $C_2$-$C_4$-cyanoalkylthio, $C_1$-$C_6$alkylsulfinyl, $C_1$-$C_6$haloalkylsulfinyl, $C_1$-$C_6$alkylsulfonyl, $C_1$-$C_6$haloalkylsulfonyl, aminosulfonyl, $C_1$-$C_2$alkylaminosulfonyl, di($C_1$-$C_2$alkyl)-aminosulfonyl, di($C_1$-$C_4$alkyl)amino, halogen, cyano, nitro, phenyl and/or by benzylthio, it being possible for phenyl and benzylthio in turn to be substituted on the phenyl ring by $C_1$-$C_3$alkyl, $C_1$-$C_3$haloalkyl, $C_1$-$C_3$alkoxy, $C_1$-$C_3$haloalkoxy, halogen, cyano or by nitro, and the substituents on the nitrogen atom in the heterocyclic ring are other than halogen; $Ra_3$ is hydrogen, $C_1$-$C_6$alk$_y$l, $C_1$-$C_6$haloalkyl, $C_2$-$C_6$alkenyl, $C_2$-$C_6$haloalkenyl, $C_2$-$C_6$alkynyl, $C_2$-$C_6$haloalkynyl, $C_3$-$C_6$cycloalkyl, $C_1$-$C_6$alkoxy, $C_1$-$C_6$haloalkoxy, $C_1$-$C_6$alkylthio, $C_1$-$C_6$alkylsulfinyl, $C_1$-$C_6$alkylsulfonyl, $C_1$-$C_6$haloalkylthio, $C_1$-$C_6$haloalkylsulfinyl, $C_1$-$C_6$haloalkylsulfonyl, $C_1$-$C_6$alkylamino, di-$C_2$-$C_6$alkylamino, $C_1$-$C_6$alkylaminosulfonyl, di-$C_2$-$C_6$alkylaminosulfonyl, phenyl, phenylthio, phenylsulfinyl, phenylsulfonyl or phenoxy, it being possible for phenyl in turn to be substituted by $C_1$-$C_3$alkyl, $C_1$-$C_3$haloalkyl, $C_1$-$C_3$alkoxy, $C_1$-$C_3$haloalkoxy, halogen, cyano or by nitro, or $Ra_3$ is -N($C_1$-$C_4$alkyl)-S-$C_1$-$C_4$alkyl, -N($C_1$-$C_4$alkyl)-SO-$C_1$-$C_4$alkyl, -N($C_1$-$C_4$alkyl)-$SO_2$-$C_1$-$C_4$alkyl, cyano, halogen, amino, $C_1$-$C_4$alkoxy-$C_1$-$C_4$alkyl, $C_1$-$C_4$alkyl-S-$C_1$-$C_4$alkyl, $C_1$-$C_4$alkyl-SO-$C_1$-$C_4$alkyl or $C_1$-$C_4$alkyl-$SO_2$-$C_1$-$C_4$alkyl;

$Ra_4$ is hydrogen, $C_1$-$C_6$alkyl, hydroxy, $C_1$-$C_6$alkoxy, $C_1$-$C_6$haloalkoxy, $C_3$-$C_6$alkenyloxy, $C_3$-$C_6$haloalkenyloxy, $C_3$-$C_6$alkynyloxy, $C_1$-$C_4$alkylcarbonyloxy, $C_1$-$C_4$alkylsulfonyloxy, tosyloxy, $C_1$-$C_4$alkylthio, $C_1$-$C_4$alkylsulfinyl, $C_1$-$C_4$alkylsulfonyl, $C_1$-$C_4$alkylamino, di-$C_1$-$C_4$alkylamino, $C_1$-$C_4$alkoxycarbonyl, $C_1$-$C_4$haloakyl, formyl, cyano, halogen, phenyl or phenoxy, it being possible for phenyl in turn to be substituted by $C_1$-$C_3$alkyl, $C_1$-$C_3$haloalkyl, $C_1$-$C_3$alkoxy, $C_1$-$C_3$haloalkoxy, halogen, cyano or by nitro;

or $Ra_4$ is a five- to ten-membered monocyclic ring system or, with $Ra_3$, annellated bicyclic ring system which may contain from 1 to 4 hetero atoms selected from nitrogen, oxygen and sulfur, the ring system, unless it is annellated, being bonded to the ring containing the substituent A directly or by way of a $C_1$-$C_4$alkylene, -CH=CH-, -C≡C-, - $CH_2O$-, -$CH_2N$($C_1$-$C_4$alkyl)-, -$CH_2S$-, -$CH_2SO$- or -$CH_2SO_2$- group, and the ring system may contain not more than two oxygen atoms and not more than two sulfur atoms, and the ring system can itself be mono-, di- or tri-substituted by $C_1$-$C_6$alkyl, $C_1$-$C_6$haloalkyl, $C_2$-$C_6$alkenyl, $C_2$-$C_6$haloalkenyl, $C_2$-$C_6$alkynyl, $C_2$-$C_6$haloalkynyl, $C_1$-$C_6$alkoxy, $C_1$-$C_6$haloalkoxy, $C_3$-$C_6$alkenyloxy, $C_3$-$C_6$alkynyloxy, $C_1$-$C_6$alkylthio, $C_1$-$C_6$haloalkylthio, $C_3$-$C_6$alkenylthio, $C_3$-$C_6$haloalkenylthio, $C_3$-$C_6$alkynylthio, $C_1$-$C_4$alkoxy-$C_1$-$C_2$alkylthio, $C_1$-$C_4$alkylcarbonyl-$C_1$-$C_2$alkylthio, $C_1$-$C_4$alkoxycarbonyl-$C_1$-$C_2$alkylthio, cyano-$C_{10}$-$C_4$alkylthio, $C_1$-$C_6$alkylsulfinyl, $C_1$-$C_6$haloalkylsulfinyl, $C_1$-$C_6$alkylsulfonyl, $C_1$-$C_6$haloalkylsulfonyl, aminosulfonyl, $C_1$-$C_2$alkylaminosulfonyl, di($C_1$-$C_2$alkyl)aminosulfonyl, di($C_1$-$C_4$alkyl)amino, halogen, cyano, nitro, phenyl and/or by benzylthio, it being possible for phenyl and benzylthio in turn to be substituted on the phenyl ring by $C_1$-$C_3$alkyl, $C_1$-$C_3$haloalkyl, $C_1$-$C_3$alkoxy, $C_1$-$C_3$haloalkoxy, halogen, cyano or by nitro, and substituents on the nitrogen atom in the heterocyclic ring are other than halogen;

$Ra_5$ is hydrogen, $C_1$-$C_6$alkyl, $C_1$-$C_6$haloalkyl, $C_2$-$C_6$alkenyl, $C_2$-$C_6$haloalkenyl, $C_2$-$C_6$alkynyl, $C_2$-$C_6$haloalkynyl; $C_3$-$C_6$cycloalkyl, $C_1$-$C_6$alkoxy, $C_1$-$C_6$haloalkoxy, $C_1$-$C_6$alkylthio, $C_1$-$C_6$alkylsulfinyl, $C_1$-$C_6$alkylsulfonyl, $C_1$-$C_6$haloalkylthio, $C_1$-$C_6$haloalkylsulfinyl, $C_1$-$C_6$haloalkylsulfonyl, $C_1$-$C_6$alkylamino, di-$C_2$-$C_6$alkylamino, $C_1$-$C_6$alkylaminosulfonyl, di-$C_2$-$C_6$alkylaminosulfonyl, phenyl, phenylthio, phenylsulfinyl, phenylsulfonyl or phenoxy, it being possible for phenyl in turn to be substituted by $C_1$-$C_3$alkyl, $C_1$-$C_3$haloalkyl, $C_1$-$C_3$alkoxy, $C_1$-$C_3$haloalkoxy, halogen, cyano or by nitro, or $Ra_5$ is -N($C_1$-$C_4$alkyl)-S-$C_1$-$C_4$alkyl, -N($C_1$-$C_4$alkyl)-SO-$C_1$-$C_4$alkyl, -N($C_1$-$C_4$alkyl)-$SO_2$-$C_1$-$C_4$alkyl, cyano, halogen, amino, $C_1$-$C_4$alkoxy-$C_1$-$C_4$alkyl, $C_1$-$C_4$alkyl-S-$C_1$-$C_4$alkyl, $C_1$-$C_4$alkyl-SO-$C_1$-$C_4$alkyl or $C_1$-$C_4$alkyl-$SO_2$-$C_1$-$C_4$alkyl, and pharmaceutically acceptable salts/N-oxides/isomers/enantiomers of those compounds.

The alkyl groups appearing in the above substituent definitions may be straight-chain or branched and are, for example, methyl, ethyl, n-propyl, isopropyl, n-butyl, sec-butyl, isobutyl or tert-butyl. Alkoxy, alkenyl and alkynyl radicals are derived from the mentioned alkyl radicals. The alkenyl and alkynyl groups may be mono- or poly-unsaturated. Alkoxy is, for example, methoxy, ethoxy, propoxy, isopropoxy, n-butoxy, isobutoxy, sec-butoxy or tert-butoxy. Alkoxycarbonyl is, for example, methoxycarbonyl, ethoxycarbonyl, propoxycarbonyl, isopropoxycarbonyl, n-butoxycarbonyl, isobutoxycarbonyl, sec-butoxycarbonyl or tert-butoxycarbonyl; preferably methoxycarbonyl or ethoxycarbonyl.

Halogen is generally fluorine, chlorine, bromine or iodine. The same is also true of halogen in conjunction with other meanings, such as haloalkyl or halophenyl.

Haloalkyl groups having a chain length of from 1 to 6 carbon atoms are, for example, fluoromethyl, difluoromethyl, trifluoromethyl, chloromethyl, dichloromethyl, trichloromethyl, 2,2,2-trifluoroethyl, 1-fluoroethyl, 2-fluoroethyl, 2-chloroethyl, 2-fluoroprop-2-yl, pentafluoroethyl, 1,1-difluoro-2,2,2-trichloroethyl, 2,2,3,3-tetrafluoroethyl and 2,2,2-trichloroethyl, pentafluoroethyl, heptafluoro-n-propyl and perfluoro-n-hexyl.

Alkenyl and alkynyl groups can be mono- or poly-unsaturated, so that alkyl, alkenyl and alkynyl chains having one or more double or triple bonds are also included. Alkenyl is, for example, vinyl, allyl, isobuten-3-yl, $CH_2$=CH-$CH_2$-CH=CH-, $CH_2$=CH-$CH_2$-$CH_2$-CH=CH- or $CH_3$-CH=CH-$CH_2$-CH=CH-. A preferred alkynyl is, for example, propargyl, and a preferred allenyl is $CH_2$=C=$CH_2$-.

An alkylene chain can also be substituted by one or more $C_1$-$C_3$alkyl groups, especially by methyl groups.

Such alkylene chains and alkylene groups are preferably unsubstituted. The same applies also to all groups containing $C_3$-$C_6$cycloalkyl, $C_3$-$C_5$oxacycloalkyl, $C_3$-$C_5$thiacycloalkyl, $C_3$-$C_4$dioxacycloalkyl, $C_3$-$C_4$dithiacycloalkyl or $C_3$-$C_4$oxathiacycloalkyl which occur, for example, also as part of oxygen- and sulfur-containing heterocyclic ring systems of the radicals $Ra_1$ and $Ra_2$.

A $C_1$-$C_4$alkylene, $C_1$-$C_4$alkenylene or $C_2$-$C_4$alkynylene bridge which may be interrupted by oxygen, -N($C_1$-$C_4$alkyl)-, sulfur, sulfinyl and/or sulfonyl, or in $X_2$ or $X_6$ in the meaning of a $C_1$-$C_6$alkylene, $C_3$-$C_6$alkenylene or $C_3$-$C_6$alkynylene chain which can be mono- or poly-substituted by halogen and/or by $X_4$ or $X_8$, and wherein the unsaturated bonds of the chain are not bonded directly to the substituent $X_1$ or $X_5$, is to be understood as being, for example, $-CH_2-$, $-CH_2CH_2-$, $-CH_2CH_2CH_2-$, $-CH_2CH_2CH_2CH_2-$, $-CH(CH_3)-$, $-CH_2CH(CH_3)-$, $-CH_2CH(CH_3)CH_2-$, $-CH_2CH(Cl)CH_2-$, $-CH_2CH(OCH_3)CH_2-$, $-CH_2O-$, $-OCH_2-$, $-CH_2OCH_2-$, $-OCH_2CH_2-$, $-OCH_2CH_2CH_2-$, $-CH_2OCH_2CH_2-$, $-CH_2OCH(CH_3)CH_2-$, $-SCH_2-$, $-SCH_2CH_2-$, $-SCH_2CH_2CH_2-$, $-CH_2S-$, $-CH_2SCH_2-$, $-CH_2S(O)CH_2-$, $-CH_2SO_2CH_2-$, $-CH_2SCH_2CH_2-$, $-CH_2S(O)CH_2CH_2-$, $-CH_2SO_2CH_2CH_2-$, $-CH_2SO_2NH-$, $-CH_2N(CH_3)SO_2CH_2CH_2-$, $-N(SO_2Me)CH_2CH_2-$, $-CH_2C(O)NH-$ or $-CH_2NHC(O)CH_2-$. A $C_2$-$C_4$alkenylene chain which may be uninterrupted or interrupted by oxygen is accordingly to be understood as being, for example, $-CH=CH-CH_2-$, $-CH=CH-CH_2CH_2-$ or $-CH=CHCH_2OCH_2-$, and a $C_2$-$C_4$alkynylene chain which may be uninterrupted or interrupted by oxygen is to be understood as being, for example, $-C\equiv C-$, $-C\equiv CCH_2-$, $-C\equiv CCH_2O-$, $-C\equiv CCH_2OCH_2-$ or $-OC\equiv CCH_2-$.

A three- to ten-membered mono- or bi-cyclic ring system $Ra_1$ or $Ra_2$, which may be interrupted once or up to three times selected from oxygen, sulfur, S(O), $SO_2$, N($Ra_6$), carbonyl and C(=$NORa_7$) and which is bonded to the carbon atom of the substituent $A_1$ or to the group $Q_1$ or $Q_2$ either directly or by way of a $C_1$-$C_4$alkylene, $C_1$-$C_4$alkenylene or $C_2$-$C_4$alkynylene bridge which may be interrupted by oxygen, -N($C_1$-$C_4$alkyl)-, sulfur, sulfinyl and/or sulfonyl, is to be understood as being, for example, 1-methyl-1H-pyrazol-3-yl, 1-ethyl-1H-pyrazol-3-yl, 1-propyl-1H-pyrazol-3-yl, 1H-pyrazol-3-yl, 1,5-dimethyl-1H-pyrazol-3-yl, 4-chloro-1-methyl-1H-pyrazol-3-yl, 1H-pyrazol-1-yl; 3-methyl-1H-pyrazol-1-yl, 3,5-dimethyl-1H-pyrazol-1-yl, 3-isoxazolyl, 5-methyl-3-isoxazolyl, 3-methyl-5-isoxazolyl, 5-isoxazolyl, 1H-pyrrol-2-yl, 1-methyl-1H-pyrrol-2-yl, 1H-pyrrol-1-yl, 1-methyl-1H-pyrrol-3-yl, 2-furanyl, 5-methyl-2-furanyl, 3-furanyl, 5-methyl-2-thienyl, 2-thienyl, 3-thienyl, 1-methyl-1H-imidazol-2-yl, 1H-imidazol-2-yl, 1-methyl-1H-imidazol-4-yl, 1-methyl-1H-imidazol-5-yl, 4-methyl-2-oxazolyl, 5-methyl-2-oxazolyl, 2-oxazolyl, 2-methyl-5-oxazolyl, 2-methyl-4-oxazolyl, 4-methyl-2-thiazolyl, 5-methyl-2-thiazolyl, 2-thiazolyl, 2-methyl-5-thiazolyl, 2-methyl-4-thiazolyl, 3-methyl-4-isothiazolyl, 3-methyl-5-isothiazolyl, 5-methyl-3-isothiazolyl, 1-methyl-1H-1,2,3-triazol-4-yl, 2-methyl-2H-1,2,3-triazol-4-yl, 4-methyl-2H-1,2,3-triazol-2-yl, 1-methyl-1H-1,2,4-triazol-3-yl, 1,5-dimethyl-1H-1,2,4-triazol-3-yl, 3-methyl-1H-1,2,4-triazol-1-yl, 5-methyl-1H-1,2,4-triazol-1-yl, 4,5-dimethyl-4H-1,2,4-triazol-3-yl, 4-methyl-4H-1,2,4-triazol-3-yl, 4H-1,2,4-triazol-4-yl, 5-methyl-1,2,3-oxadiazol-4-yl, 1,2,3-oxadiazol-4-yl, 3-methyl-1,2,4-oxadiazol-5-yl, 5-methyl-1,2,4-oxadiazol-3-yl, 4-methyl-3-furazanyl, 3-furazanyl, 5-methyl-1,2,4-oxadiazol-2-yl, 5-methyl-1,2,3-thiadiazol-4-yl, 1,2,3-thiadiazol-4-yl, 3-methyl-1,2,4-thiadiazol-5-yl, 5-methyl-1,2,4-thiadiazol-3-yl, 4-methyl-1,2,5-thiadiazol-3-yl, 5-methyl-1,3,4-thiadiazol-2-yl, 1-methyl-1H-tetrazol-5-yl, 1H-tetrazol-5-yl, 5-methyl-1H-tetrazol-1-yl, 2-methyl-2H-tetrazol-5-yl, 2-ethyl-2H-tetrazol-5-yl, 5-methyl-2H-tetrazol-2-yl, 2H-tetrazol-2-yl, 2-pyridinyl, 6-methyl-2-pyridinyl, 4-pyridinyl, 3-pyridinyl, 6-methyl-3-pyridazinyl, 5-methyl-3-pyridazinyl, 3-pyridazinyl, 4,6-dimethyl-2-pyrimidinyl, 4-methyl-2-pyrimidinyl, 2-pyrimidinyl, 2-methyl-4-pyrimidinyl, 2-chloro-4-pyrimidinyl, 2,6-dimethyl-4-pyrimidinyl, 4-pyrimidinyl, 2-methyl-5-pyrimidinyl, 6-methyl-2-pyrazinyl, 2-pyrazinyl, 4,6-dimethyl-1,3,5-triazin-2-yl, 4,6-dichloro-1,3,5-triazin-2-yl, 1,3,5-triazin-2-yl, 4-methyl-1,3,5-triazin-2-yl, 3-methyl-1,2,4-triazin-5-yl, 3-methyl-1,2,4-triazin-6-yl,

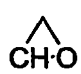 , 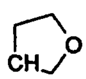 , 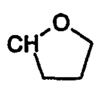 , 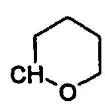 , 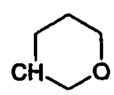 ,

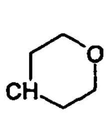 , 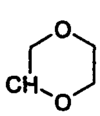 , 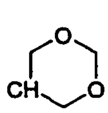 , 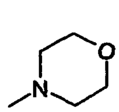 , 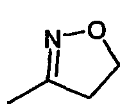 , 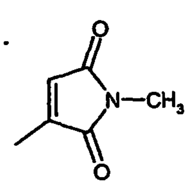 , 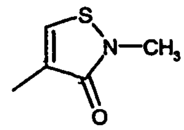 ,

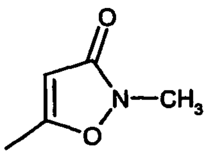, 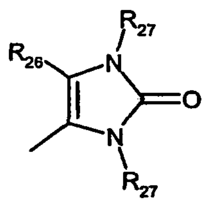, 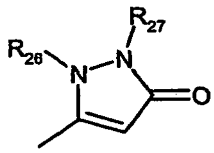, 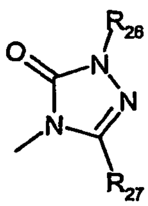, 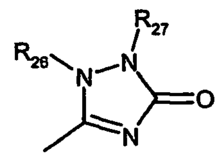,

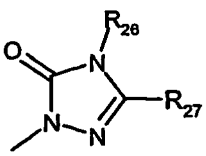, 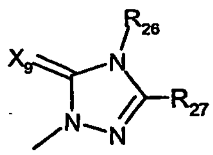, 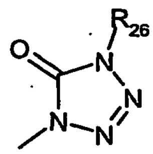, 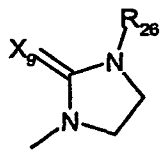, 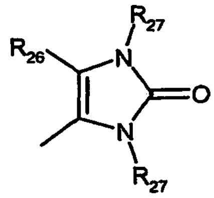,

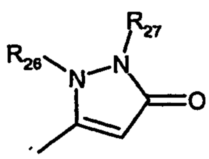 or 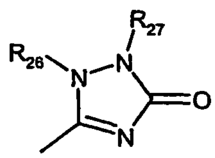,

, wherein each $R_{26}$ is methyl, each $R_{27}$ independently is hydrogen, $C_1$-$C_3$alkyl, $C_1$-$C_3$alkoxy, $C_1$-$C_3$alkylthio or trifluoromethyl, and $X_9$ is oxygen or sulfur.

A further annellated (fused-on), monocyclic or bicyclic ring system which is formed; for example, by two adjacent substituents $Ra_1$ and $Ra_2$ or $Ra_1$ and $Ra_5$ and which is uninterrupted or interrupted once or up to three times selected from oxygen, sulfur, S(O), $SO_2$ -N($Ra_6$)-, carbonyl and C(=NO$Ra_7$) and which may be additionally substituted by one or more substituents is to be understood as being, for example, an annellated, bidentate ring system of formula

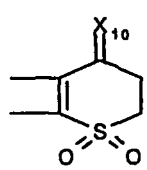, 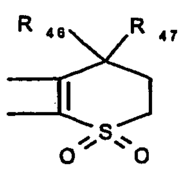, 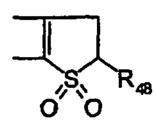,

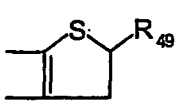, 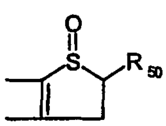, 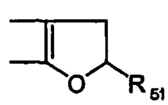, 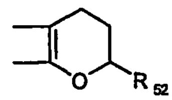, 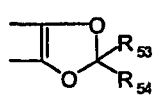, 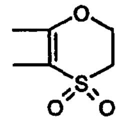,

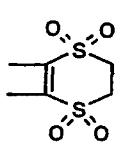, 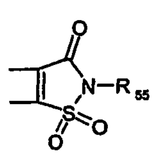, 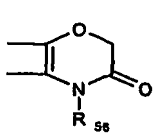, 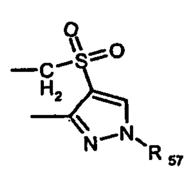 or 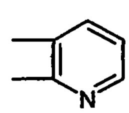,

wherein especially $R_{46}$ is hydrogen, halogen, $C_1$-$C_4$alkyl, $C_1$-$C_4$haloalkyl, $C_1$-$C_4$alkoxy or $C_1$-$C_4$alkylthio; $R_{47}$ is hydrogen, halogen, $C_1$-$C_4$alkyl or $C_1$-$C_4$alkoxy; and $R_{50}$, $R_{51}$, $R_{52}$, $R_{53}$, $R_{54}$, $R_{55}$, $R_{56}$, $R_{57}$, $R_{58}$ and $R_{59}$ are hydrogen or $C_1$-$C_4$alkyl; and $X_{10}$ is oxygen or NO$R_{59}$.

A number of HPPD inhibitors of formula I are described within the art.

In a particular embodiment of the invention the HPPD inhibitor comprises the compound of formula I wherein:

T is $T_1$;
$R_1$ and $R_2$ are hydrogen;
A is $C_1$-$C_2$alkylene;
D and E together are $C_2$-$C_3$alkylene;
Q is $Q_1$, wherein
$A_1$ is methine, $CRa_1$ or N-(O)p, but preferably =N-(O)p;
p is 0;
$Ra_1$ is hydrogen, $C_1$-$C_6$alkyl, hydroxy, $C_1$-$C_6$alkoxy, $C_1$-$C_6$haloalkoxy, $C_3$-$C_6$alkenyloxy, $C_3$-$C_6$haloalkenyloxy, $C_3$-$C_6$alkynyloxy, $C_1$-$C_4$alkylcarbonyloxy, $C_1$-$C_4$alkylsulfonyloxy, tosyloxy, $C_1$-$C_4$alkylthio, $C_1$-$C_4$alkylsulfinyl, $C_1$-$C_4$alkylsulfonyl, $C_1$-$C_4$alkylamino, di-$C_1$-$C_4$alkylamino, $C_1$-$C_4$alkoxycarbonyl, $C_1$-$C_4$haloalkyl, formyl, cyano, halogen, phenyl or phenoxy; it being possible for phenyl in turn to be substituted by $C_1$-$C_3$alkyl, $C_1$-$C_3$haloalkyl, $C_1$-$C_3$alkoxy, $C_1$-$C_3$haloalkoxy, halogen, cyano or by nitro;
$Ra_2$ is hydrogen, $C_1$-$C_6$alkyl, $C_1$-$C_6$haloalkyl, $C_2$-$C_6$alkenyl, $C_2$-$C_6$haloalkenyl, vinyl substituted by $C_1$-$C_2$alkoxycarbonyl or by phenyl, $C_2$-$C_6$alkynyl, $C_2$-$C_6$haloalkynyl, ethynyl substituted by trimethylsilyl, hydroxy, $C_1$-$C_2$alkoxy, $C_1$-$C_2$alkoxycarbonyl or by phenyl, $C_3$-$C_6$allenyl, $C_3$-$C_6$cycloalkyl, halo-substituted $C_3$-$C_6$cycloalkyl, $C_1$-$C_6$alkoxy, $C_3$-$C_6$alkenyloxy, $C_3$-$C_6$alkynyloxy, $C_1$-$C_6$haloalkoxy, $C_3$-$C_6$haloalkenyloxy, cyano-$C_1$-$C_4$alkoxy, $C_1$-$C_4$alkoxy-$C_1$-$C_4$alkoxy, $C_1$-$C_4$alkylthio-$C_1$-$C_4$alkoxy, $C_1$-$C_4$alkylsulfinyl-$C_1$-$C_4$alkoxy, $C_1$-$C_4$alkylsulfonyl-$C_1$-$C_4$alkoxy, $C_1$-$C_4$alkoxycarbonyl-$C_1$-$C_4$alkoxy, $C_1$-$C_6$alkylthio, $C_1$-$C_6$alkylsulfinyl, $C_1$-$C_6$alkylsulfonyl, $C_1$-$C_6$haloalkylthio, $C_1$-$C_6$haloalkylsulfinyl, $C_1$-$C_6$haloalkylsulfonyl, $C_1$-$C_4$alkoxycarbonyl-$C_1$-$C_4$alkylthio, $C_1$-$C_4$alkoxycarbonyl-$C_1$-$C_4$alkylsulfinyl, $C_1$-$C_4$alkoxycarbonyl-$C_1$-$C_4$alkylsulfonyl, benzyl-S-, benzyl-SO-, benzyl-$SO_2$-, $C_1$-$C_6$alkylamino, di-$C_2$-$C_6$alkylamino, $C_1$-$C_6$alkylaminosulfonyl, di($C_1$-$C_6$alkylamino)sulfonyl, benzyloxy, benzyl, phenyl, phenoxy, phenylthio, phenylsulfinyl or phenylsulfonyl, it being possible for the phenyl-containing groups in turn to be substituted by $C_1$-$C_3$alkyl, $C_1$-$C_3$haloalkyl, $C_1$-$C_3$alkoxy, $C_1$-$C_3$haloalkoxy, halogen, cyano or by nitro, or $Ra_2$ is OS-$C_1$-$C_4$alkyl, OSO-$C_1$-$C_4$alkyl, $OSO_2$-$C_1$-$C_4$alkyl, OS-$C_1$-$C_4$haloalkyl, OSO-$C_1$-$C_4$haloalkyl, $OSO_2$-$C_1$-$C_4$haloalkyl, N($C_1$-$C_4$alkyl)-S-$C_1$-$C_4$alkyl, N($C_1$-$C_4$alkyl)-SO-$C_1$-$C_4$alkyl, N($C_1$-$C_4$alkyl)-$SO_2$-$C_1$-$C_4$alkyl, cyano, carbamoyl, $C_1$-$C_4$alkoxycarbonyl, formyl, halogen, rhodano, amino, hydroxy-$C_1$-$C_4$alkyl, $C_1$-$C_4$alkoxy-$C_1$-$C_4$alkyl, $C_1$-$C_4$alkyl-S-$C_1$-$C_4$alkyl, $C_1$-$C_4$alkyl-SO-$C_1$-$C_4$alkyl, $C_1$-$C_4$alkyl-$SO_2$-$C_1$-$C_4$alkyl, cyano-$C_1$-$C_4$alkyl, $C_1$-$C_6$alkylcarbonyloxy-$C_1$-$C_4$alkyl, $C_1$-$C_4$alkoxycarbonyl-$C_1$-$C_4$alkyl, $C_1$-$C_4$alkoxycarbonyloxy-$C_1$-$C_4$alkyl, $C_1$-$C_4$-rhodano-$C_1$-$C_4$alkyl, benzoyloxy-$C_1$-$C_4$alkyl, $C_2$-$C_6$oxiranyl, $C_1$-$C_4$alkylamino-$C_1$-$C_4$alkyl, di($C_1$-$C_4$alkyl)amino-$C_1$-$C_4$alkyl, $C_1$-$C_{12}$alkylthiocarbonyl-$C_1$-$C_4$alkyl or formyl-$C_1$-$C_4$alkyl, or $Ra_2$ is the group -$X_1$-$X_3$ or the group -$X_2$-$X_1$-$X_3$; wherein $X_1$, $X_2$ and $X_3$ are as defined above;
$Ra_3$ and $Ra_4$ are hydrogen and $Ra_5$ is as defined above.

In a still further embodiment the invention the HPPD inhibitor comprises a compound of formula I wherein:

T is $T_1$;
$R_1$ and $R_2$ are hydrogen, A is methylene, D and E together are ethylene, $A_1$ is =N-$(O)_p$; wherein p is 0;
Q is $Q_1$, $Ra_3$ and $Ra_4$ are hydrogen, $Ra_5$ is $C_1$-$C_3$haloalkyl, especially trifluoromethyl, and $Ra_2$ is $C_1$-$C_4$alkoxy-$C_1$-$C_4$alkoxy-$C_1$-$C_4$alkyl, especially methoxyethoxymethyl.

HPPD inhibiting compounds are well known in the art and there are numerous tests that can be employed to identify the capacity of a test compound to inhibit HPPD. For example, *in vitro* screening assays as described in the examples of the present application may be use or alternative *in vitro* screening methods can be employed such as the method described in example 11 of WO02/46387 wherein a known HPPD enzyme is selected and a test inhibitor compound is applied.

In a still further embodiment of the invention the HPPD inhibitor or precursor is a compound having the structure depicted in Table A below.

Table A.

| | Compound Number | Structure: | |
|---|---|---|---|
| | 3.01 | 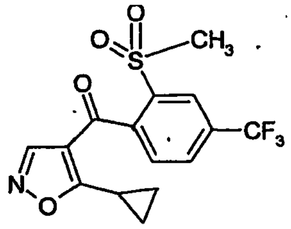 | |
| | 3.02 | 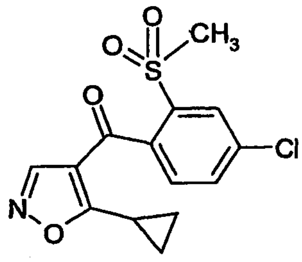 | |
| | 3.03 | 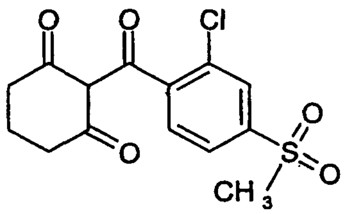 | |
| | 3.04 | 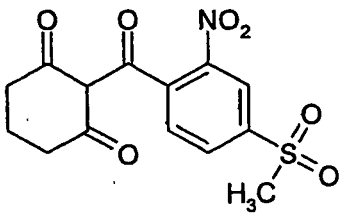 | |
| | 3.05 | 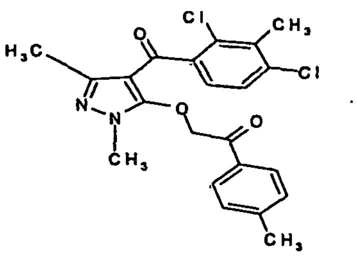 | |
| | 3.06 | 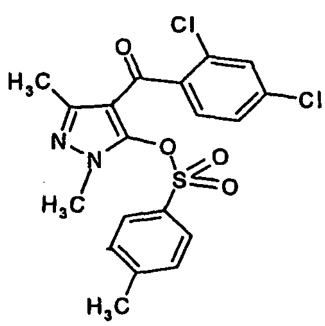 | |

(continued)

| | Compound Number | Structure: | |
|---|---|---|---|
| | 3.07 | 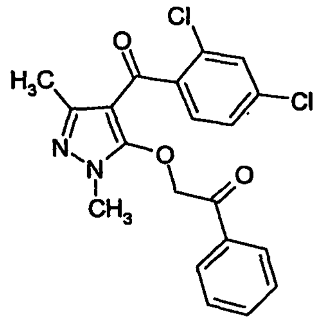 | |
| | 3.08 | 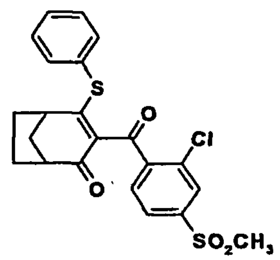 | |
| | 3.09 | 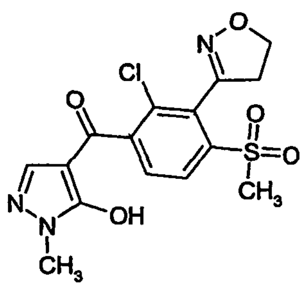 | |
| | 3.10 | 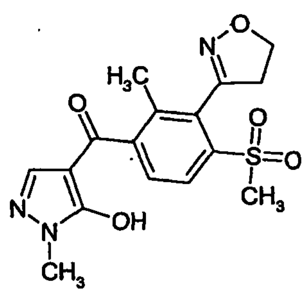 | |
| | 3.11 | 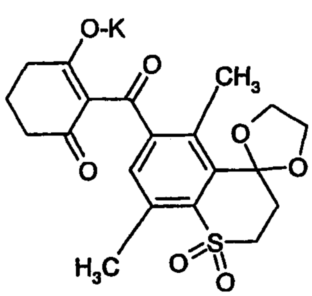 | and the free acid thereof |

(continued)

| | Compound Number | Structure: | |
|---|---|---|---|
| | 3.12 | 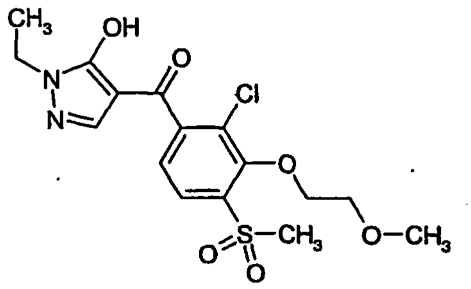 | |
| | 3.13 | 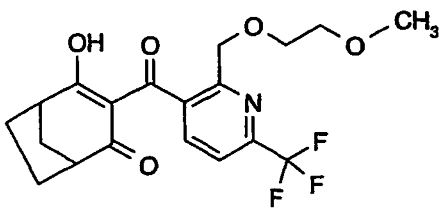 | |
| | 3.14 | 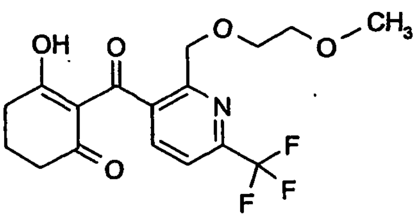 | |
| | 3.15 | 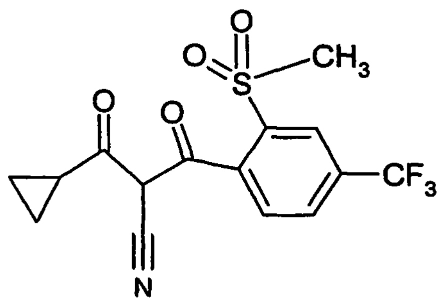 | |
| | 3.16 | 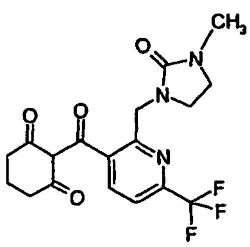 | |
| | 3.17 | 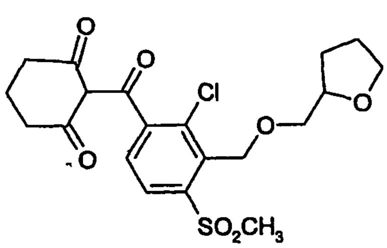 | |

(continued)

| | Compound Number | Structure: | |
|---|---|---|---|
| | 3.18 | 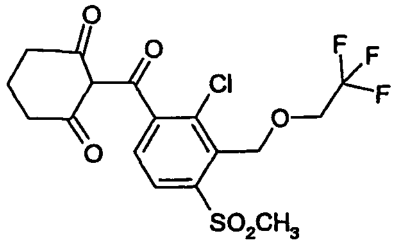 | |
| | 3.19 | 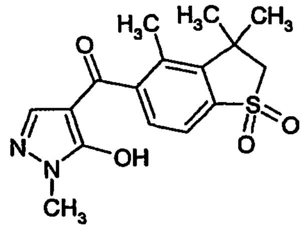 | |
| | 3.20 | 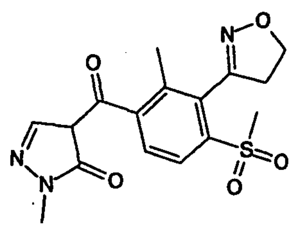 | |
| | 3.21 | 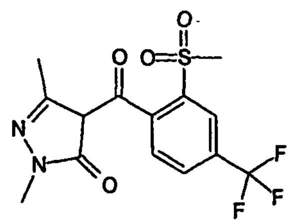 | |
| | 3.22 | 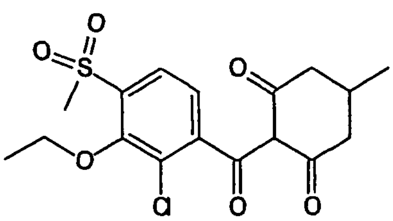 | |
| | 3.23 | 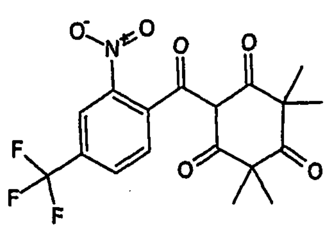 | |

(continued)

| | Compound Number | Structure: | |
|---|---|---|---|
| | 3.24 | 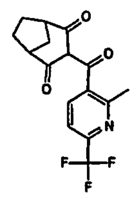 | |
| | 3.25 | 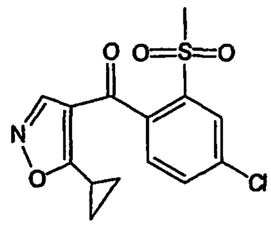 | |
| | 3.26 | 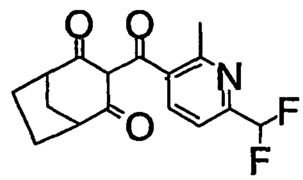 | |

In addition to the compounds described herein, it is also possible to use a compound which is a precursor to an HPPD inhibiting compound.

A "precursor" is a compound which itself is not an HPPD inhibitor but is metabolised to produce an HPPD inhibitor for use in accordance with the present invention. For example the compound depicted as compound No. 3.01 in Table A above is a precursor to the compound depicted as compound No. 3.15.

Thus, throughout this specification, "HPPD inhibitor" includes those compounds which are capable of inhibiting HPPD in animals and any precursor compound thereof which is capable of being metabolised in the animal to produce the HPPD inhibiting compound.

The present invention further provides the use as described above wherein said disease is treated. In a particular embodiment said treatment includes retarding the progression of said disease. In a further embodiment said treatment ameliorates the symptoms of said disease.

The present invention still further provides the use as described above wherein said disease is prevented.

The present invention still further provides the use as described above wherein said animal is a human being.

The present invention still further provides the use as described above wherein said animal or said human being is suffering from a neurodegenerative disease.

The present invention still further provides the use as described above wherein said disease is Parkinson's disease.

The term "Parkinson's disease" throughout this specification includes: idiopathic Parkinson's disease; early-onset Parkinson's disease; post-encephalitic parkinsonism; drug-induced Parkinson's disease; toxin-induced Parkinson's disease; post-traumatic parkinsonism; dopa-responsive dystonia; Machado Joseph disease (also referred to as spinocerebellar ataxia Type 3); multiple system atrophy (which includes olivopontocerebellar atrophy, striatonigral disease and Shy-Drager syndrome); progressive subnuclear palsy; and vascular parkinsonism. In a particular embodiment of the invention the term "Parkinson's disease" means idiopathic Parkinson's disease.

In a further aspect of the invention there is provided the use of a compound of formula 1 or a pharmaceutically acceptable salt thereof, in the manufacture of a medicament for use in the treatment of a neurodegenerative disease. In a particular embodiment of the invention said neurodegenerative disease is Parkinson's disease.

In a still further aspect of the invention there is provided the use of any one of compounds depicted as 2, 3.01, 3.11, 3.12, 3.13, 3.15, 3.18, 3.20, 3.21, 3.22, 3.23, 3.24, 3.25 and 3.26 or a pharmaceutically acceptable salt thereof, in the manufacture of a medicament for use in the treatment of a neurodegenerative disease. In a particular embodiment said compound is any one of compounds depicted as 2, 3.01, 3.11, 3.12, 3.15, 3.18, 3.20, 3.23 and 3.24. In a particular embodiment of the invention said neurodegenerative disease is Parkinson's disease.

The present invention still further provides the use as described above wherein said medicament is administered in combination with an anti-inflammatory agent.

The present invention still further provides the use as described above wherein said medicament comprises an anti-inflammatory agent.

The present invention still further provides the use as described above wherein said medicament comprises a first HPPD inhibitor and a further HPPD inhibitor and wherein said first inhibitor is different from said further inhibitor. In a particular embodiment said first and further HPPD inhibitor is selected from an inhibitor described above. In a still further embodiment said first inhibitor is any one of compounds depicted as 2, 3.01, 3.11, 3.12, 3.13, 3.15, 3.18, 3.20, 3.21, 3.22, 3.23, 3.24, 3.25 and 3.26 or a pharmaceutically acceptable salt thereof. In a still further embodiment said first inhibitor is any one of the compounds depicted as 2, 3.01, 3.11, 3.12, 3.15, 3.18, 3.20, 3.23 and 3.24 or a pharmaceutically acceptable salt thereof.

The present invention still further provides the use as described above wherein said medicament comprises a dopamine agonist. In a particular embodiment said agonist comprises a compound selected from the group consisting of: Pramipexole; Cabergoline; Pergolide; and Ropinirole. In a particular embodiment said agonist may be administered separately to the medicament comprising said HPPD inhibitor.

The present invention still further provides the use as described above wherein said medicament comprises levodopa. In a particular embodiment said levodopa may be administered separately to the medicament comprising said HPPD inhibitor.

The present invention still further provides the use as described above wherein said medicament comprises levodopa and a decarboxylase inhibitor. In a particular embodiment said levodopa and a decarboxylase inhibitor combination comprises Carbidopa and Levodopa. In a still further embodiment said levodopa and a decarboxylase inhibitor combination comprises Levodopa and Benserazide. In a particular embodiment said levodopa and a decarboxylase inhibitor may be administered separately to the medicament comprising said HPPD inhibitor.

The present invention still further provides the use as described above wherein said medicament comprises Entacapone. In a particular embodiment said medicament comprises Carbidopa, Levodopa and Entacapone. In a particular embodiment said Entacapone or said Carbidopa, Levodopa and Entacapone may be administered separately to the medicament comprising said HPPD inhibitor.

The present invention still further provides the use as described above wherein said medicament comprises a catechol-O-methyl transferase (COMT) inhibitor. In a particular embodiment said COMT inhibitor comprises Tolcapone. In a particular embodiment said COMT inhibitor may be administered separately to the medicament comprising said HPPD inhibitor.

The present invention still further provides the use as described above wherein said medicament comprises a monoamine oxidase (MAO) inhibitor. In a particular embodiment said MAO inhibitor may be administered separately to the medicament comprising said HPPD inhibitor.

The present invention still further provides the use as described above wherein said medicament comprises an anti-dyskinesia agent. In a particular embodiment said anti-dyskinesia agent may be administered separately to the medicament comprising said HPPD inhibitor.

The present invention still further provides the use as described above wherein said medicament comprises a decarboxylase inhibitor.

The present invention still further provides the use as described above wherein said medicament comprises a neuroprotectant.

The present invention still further provides the use as described above wherein said medicament comprises an adenosine (A2a) receptor antagonist. In a particular embodiment said antagonist comprises istradefylline.

The present invention still further provides the use as described above wherein said medicament comprises a HPPD inhibitor (or a precursor thereof) and at least one of the following: (a) a dopamine agonist; (b) levodopa; (c) levodopa and a decarboxylase inhibitor; (d) levodopa and a decarboxylase inhibitor and Entacapone; (e) a catechol-O-methyl transferase inhibitor; (f) a monoamine oxidase inhibitor; (g) an anti-dyskinesia agent; (h) an anti-inflammatory agent; (i) a further HPPD inhibitor (or a precursor thereof); (j) a decarboxylase inhibitor; (k) a neuroprotectant; (1) an adenosine (A2a) receptor antagonist; (m) istradefylline.

In a still further aspect of the invention there is provided the compound depicted as 3.01, 3.11, 3.12, 3.13, 3.15, 3.18, 3.20, 3.21, 3.22, 3.23, 3.24, 3.25 and 3.26 or a pharmaceutically acceptable salt thereof for use as pharmaceutical agent.

In a still further aspect of the invention there is provided the compound depicted as 3.01, 3.11, 3.12, 3.13, 3.15, 3.18, 3.20, 3.21, 3.22, 3.23, 3.24, 3.25 and 3.26 or a pharmaceutically acceptable salt thereof, for use as an inhibitor of the catalytic activity of 4-hydroxyphenylpyruvate dioxygenase in a patient suffering from Parkinson's disease.

In a still further aspect of the invention there is provided a compound depicted as compound depicted as 3.01 to 3.26 inclusive or a pharmaceutically acceptable salt thereof, for use as a medicament.

In a particular embodiment there is provided a compound depicted as 3.01, 3.02, 3.03, 3.04, 3.05, 3.06, 3.07,

3.08, 3.09, 3.10, 3.11, 3.12, 3.13, 3.14, 3.15, 3.16, 3.17, 3.18, 3.19, 3.20, 3.21, 3.22, 3.23, 3.24, 3.25, 3.26 or a pharmaceutically acceptable salt thereof, for use as a medicament.

In a particular embodiment of the invention there is provided a compound depicted as 3.01, 3.11, 3.12, 3.13, 3.15, 3.18, 3.20, 3.21, 3.22, 3.23, 3.24, 3.25 and 3.26 or a pharmaceutically acceptable salt thereof, for use as a medicament.

In a further embodiment there is provided the use of a precursor of the compound depicted as 3.01, 3.11, 3.12, 3.13, 3.15, 3.18, 3.20, 3.21, 3.22, 3.23, 3.24, 3.25 and 3.26 for use as a medicament.

In a still further aspect of the invention there is provided a kit comprising a pharmaceutically effective amount of a HPPD inhibitor other than 2-(2-Nitro-4-Trifluoromethylbenzoyl)-1,3-Cyclohexanedione and a means for the delivery thereof to an animal.

In a still further aspect of the invention there is provided a kit comprising a pharmaceutically effective amount of a HPPD inhibitor and an anti-inflammatory agent and a means for the delivery thereof to an animal.

In a still further aspect of the invention there is provided a kit comprising a pharmaceutically effective amount of a first HPPD inhibitor and a further HPPD inhibitor and wherein said first inhibitor is different from said further inhibitor. In a particular embodiment said first and further HPPD inhibitor is selected from an inhibitor described above. In a still further embodiment said first inhibitor comprises 2-(2-Nitro-4-Trifluoromethylbenzoyl)-1,3-Cyclohexanedione (compound 2). In a still further embodiment said first inhibitor comprises the depicted as 3.01, 3.11, 3.12, 3.13, 3.15, 3.18, 3.20, 3.21, 3.22, 3.23, 3.24, 3.25 and 3.26 as described above.

In a still further aspect of the invention there is provided a kit comprising a pharmaceutically effective amount of a HPPD inhibitor and a pharmaceutically effective amount of a dopamine agonist and a means for the delivery thereof to an animal.

In a still further aspect of the invention there is provided a kit comprising a pharmaceutically effective amount of a HPPD inhibitor and a pharmaceutically effective amount of levodopa and a means for the delivery thereof to an animal.

In a still further aspect of the invention there is provided a kit comprising a pharmaceutically effective amount of a HPPD inhibitor and a pharmaceutically effective amount of levodopa and a decarboxylase inhibitor and a means for the delivery thereof to an animal.

In a still further aspect of the invention there is provided a kit comprising a pharmaceutically effective amount of a HPPD inhibitor and a pharmaceutically effective amount of levodopa and a decarboxylase inhibitor and Entacapone and a means for the delivery thereof to an animal.

In a still further aspect of the invention there is provided a kit comprising a pharmaceutically effective amount of a HPPD inhibitor and a pharmaceutically effective amount of a catechol-O-methyl transferase inhibitor and a means for the delivery thereof to an animal.

In a still further aspect of the invention there is provided a kit comprising a pharmaceutically effective amount of a HPPD inhibitor and a pharmaceutically effective amount of a monoamine oxidase inhibitor and a means for the delivery thereof to an animal.

In a still further aspect of the invention there is provided a kit comprising a pharmaceutically effective amount of a HPPD inhibitor and a pharmaceutically effective amount of and an anti-dyskinesia agent and a means for the delivery thereof to an animal.

In a still further aspect of the invention there is provided a kit comprising a pharmaceutically effective amount of a HPPD inhibitor and a pharmaceutically effective amount of a decarboxylase inhibitor and a means for the delivery thereof to an animal.

In a still further aspect of the invention there is provided a kit comprising a pharmaceutically effective amount of a HPPD inhibitor and a pharmaceutically effective amount of a neuroprotectant and a means for the delivery thereof to an animal.

In a still further aspect of the invention there is provided a kit comprising a pharmaceutically effective amount of a HPPD inhibitor and a pharmaceutically effective amount of an adenosine (A2a) receptor antagonist and a means for the delivery thereof to an animal.

In a still further aspect of the invention there is provided a kit comprising a pharmaceutically effective amount of a HPPD inhibitor and a pharmaceutically effective amount of istradefylline and a means for the delivery thereof to an animal.

In a still further aspect of the invention there is provided a kit comprising a pharmaceutically effective amount of a HPPD inhibitor and a pharmaceutically effective amount of at least one of the following: (a) a dopamine agonist; (b) levodopa; (c) levodopa and a decarboxylase inhibitor; (d) levodopa and a decarboxylase inhibitor and Entacapone; (e) a catechol-O-methyl transferase inhibitor; (f) a monoamine oxidase inhibitor; (g) an anti-dyskinesia agent; (h) an anti-inflammatory agent; (i) a further HPPD inhibitor (or a precursor thereof); (j) a decarboxylase inhibitor; (k) a neuroprotectant; (1) an adenosine (A2a) receptor antagonist; (m) istradefylline; and, a means for the delivery thereof to an animal.

In a particular embodiment there is provided a kit as described above wherein said animal is a human being.

As described above, said HPPD inhibitor may comprise a precursor compound.

In a still further aspect of the invention there is provided a pharmaceutical composition comprising as an active ingredient any one of the compounds depicted as compound 3.01 to 3.26 inclusive or a pharmaceutically acceptable salt thereof, together with a pharmaceutically acceptable diluent or carrier.

In a still further aspect of the invention there is provided a pharmaceutical composition comprising as an active ingredient any one of the compounds depicted as compound 3.01, 3.11, 3.12, 3.13, 3.15, 3.18, 3.20, 3.21, 3.22, 3.23, 3.24, 3.2 and 3.26 or a pharmaceutically acceptable salt thereof, together with a pharmaceutically acceptable diluent or carrier. In a particular embodiment said pharmaceutical composition comprises as an active ingredient any one of the compounds depicted as compound 3.01, 3.11, 3.12, 3.15, 3.18, 3.20, 3.23 and 3.24 or a pharmaceutically acceptable salt thereof, together with a pharmaceutically acceptable diluent or carrier.

In a particular embodiment of the invention said pharmaceutical composition is in a form suitable for oral or parenteral administration. In a further embodiment of the invention said composition is in palatable form suitable for oral administration selected from the group consisting of: tablets; lozenges; hard capsules; aqueous suspensions; oily suspensions; emulsions; dispersible powders; dispersible granules; syrups and elixirs.

In a still further embodiment of the invention said composition is intended for oral use and is in the form of hard or soft gelatin capsules.

In a still further embodiment of the invention said composition is in a form suitable for parenteral administration.

In a still further embodiment of the invention there is provided a pharmaceutical which comprises a composition as described above in combination with a further HPPD inhibitor which is different from the compound depicted as compound 3.01, 3.11, 3.12, 3.13, 3.15, 3.18, 3.20, 3.21, 3.22, 3.23, 3.24, 3.25 and 3.26.

In a still further embodiment of the invention there is provided a pharmaceutical which comprises a composition as described above in combination with a further HPPD inhibitor which further inhibitor is selected from the compounds depicted as compound 3.01, 3.11, 3.12, 3.13, 3.15, 3.18, 3.20, 3.21, 3.22, 3.23, 3.24, 3.25 and 3.26.

In a still further embodiment of the invention there is provided a pharmaceutical which comprises compound 2 and a further HPPD inhibitor. In a still further embodiment said further inhibitor is a compound depicted as compound 3.01, 3.11, 3.12, 3.13, 3.15, 3.18, 3.20, 3.21, 3.22, 3.23, 3.24, 3.25 and 3.26.

In a still further aspect of the invention there is provided a pharmaceutical composition comprising a pharmaceutically effective amount of a HPPD inhibitor and a pharmaceutically effective amount of a dopamine agonist optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of levodopa optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of levodopa and a decarboxylase inhibitor optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of a catechol-O-methyl transferase inhibitor optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of a monoamine oxidase inhibitor optionally together with a pharmaceutically acceptable diluent or carrier.

In a still further aspect of the invention there is provided a pharmaceutical composition comprising a pharmaceutically effective amount of a first HPPD inhibitor and a pharmaceutically effective amount of a further HPPD inhibitor wherein said first inhibitor is different from said further inhibitor optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of a decarboxylase inhibitor optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of a neuroprotectant optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of an adenosine (A2a) receptor antagonist optionally together with a pharmaceutically acceptable diluent or carrier.

In a still further aspect of the invention there is provided a pharmaceutical composition comprising a pharmaceutically effective amount of a HPPD inhibitor and a pharmaceutically effective amount of istradefylline optionally together with a pharmaceutically acceptable diluent or carrier.

In a particular embodiment of the invention there is provided a pharmaceutical composition as described above

wherein said HPPD inhibitor comprises pharmaceutical composition comprising as an active ingredient any one of the compounds depicted as compound 3.01, 3.11, 3.12, 3.13, 3.15, 3.18, 3.20, 3.21, 3.22, 3.23, 3.24, 3.25 and 3.26 or a pharmaceutically acceptable salt thereof, optionally together with a pharmaceutically acceptable diluent or carrier.

The compositions of such HPPD inhibitors for use in the invention may be in various conventional forms well know in the pharmaceutical art and which are especially adapted for pharmaceutical purposes that is for administration to man and other warm-blooded animals.

Thus, they may be in a palatable form suitable for oral use (for example as tablets, lozenges, hard or soft capsules, aqueous or oily suspensions, emulsions, dispersible powders or granules, syrups or elixirs), or for parenteral administration (for example as a sterile aqueous or oily solution for intravenous, subcutaneous, intramuscular or intravascular dosing).

The compositions of the invention may be obtained by conventional procedures using conventional pharmaceutical excipients, well known in the art.

Thus, compositions intended for oral use will normally contain, for example, at least one or more colouring, sweetening, flavouring and/or preservative agents and may be in the form of hard gelatin capsules in which the active ingredient is mixed with an inert solid diluent, for example, calcium carbonate, calcium phosphate or kaolin, Compositions for oral use may also be in the form of soft gelatin capsules in which the active ingredient is mixed with water or an oil such as arachis oil, liquid paraffin or olive oil.

Suitable pharmaceutically acceptable excipients for use in tablet formulations include, for example, inert diluents such as lactose, sodium carbonate, calcium phosphate or calcium carbonate, granulating and disintegrating agents such as corn starch or alginic acid; binding agents such as gelatin or starch; lubricating agents such as magnesium stearate, stearic acid or talc; preservative agents such as ethyl or propyl p-hydroxybenzoate, and anti-oxidants, such as ascorbic acid.

Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Aqueous suspensions will generally contain the active ingredient in finely powdered form together with one or more pharmaceutically acceptable suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan mono-oleate.

Aqueous suspensions will also typically contain one or more preservatives (such as ethyl or propyl p-hydroxybenzoate, anti-oxidants (such as ascorbic acid), colouring agents, normally together with a flavouring and/or sweetening agent (such as sucrose, saccharin or aspartame).

Oily suspensions may be formulated by suspending the active ingredient in a vegetable oil (such as arachis oil, olive oil, sesame oil or coconut oil) or in a mineral oil (such as liquid paraffin).

The oily suspensions may also contain a thickening agent such as beeswax, hard paraffin or cetyl alcohol.

Sweetening agents such as those set out above, and flavouring agents may be added to provide a palatable oral preparation.

These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives.

Suitable dispersing or wetting agents and suspending agents are exemplified by those already mentioned above.

Additional pharmaceutically acceptable excipients such as sweetening, flavouring and colouring agents, will generally also be present.

The pharmaceutical compositions of the invention may also be in the form of oil-in-water emulsions.

The oily phase may be a vegetable oil, such as olive oil or arachis oil, or a mineral oil, such as for example liquid paraffin or a mixture of any of these.

Suitable emulsifying agents may be, for example, naturally-occurring gums such as gum acacia or gum tragacanth, naturally-occurring phosphatides such as soya bean, lecithin, or esters or partial esters derived from fatty acids and hexitol anhydrides (for example sorbitan monooleate) and condensation products of the said partial esters with ethylene oxide such as polyoxyethylene sorbitan monooleate.

The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above.

A sterile injectable preparation may also be a sterile injectable solution or suspension in a non-toxic parenterally-acceptable diluent or solvent, for example a solution in 1,3-butanediol.

Dosage

The amount of active ingredient that is combined with one or more excipients to produce a single dosage form will necessarily vary depending upon the host treated and the particular route of administration.

Generally, a formulation intended for oral administration to humans will generally contain for example from 0.01mg to 10mg of active agent per Kg of bodyweight combined with an appropriate and convenient amount of excipients.

Dosage unit forms will generally contain about 0.1 mg to about 500 mg of an active ingredient.

More specifically, a formulation comprising compound 2, for example, intended for oral administration to humans will generally contain for example from 0.01mg to 1mg of active agent per Kg of bodyweight combined with an appropriate and convenient amount of excipients.

Dosage unit forms for a formulation comprising compound 2 will generally contain about 0.1 mg to about 100 mg of an active ingredient.

However, it will be readily understood that it may be necessary to vary the dose of the active ingredient administered in accordance with well known medical practice in order to take account of the nature and severity of the condition or disease under treatment, any concurrent therapy, and of the age, weight, genotype and sex of the patient receiving treatment.

Generally, in therapeutic use, it is envisaged that a composition according to the invention would be administered so that a dose of the HPPD inhibitor (or of an equivalent amount of a pharmaceutically acceptable salt thereof) is received which is generally in the range 0.00002 to 10 mg/kg/day, or 0.001 to 500 mg/day more specifically, 0.05-10mg/day and 0.1-5mg/day or 0.01 to 10 mg of active agent per Kg of bodyweight daily given if necessary in divided doses.

More specifically, for a composition comprising compound 2, in therapeutic use, it is envisaged that a composition according to the invention would,be administered so that a dose of the HPPD inhibitor (or of an equivalent amount of a pharmaceutically acceptable salt thereof) is received which is generally in the range 0.0002 to 1 mg/kg/day, or 0.01 to 100 mg/day. More specifically, from between 0.05 to 10mg/day and 0.1 to 5mg/day or 0.01 to 1mg of active agent per Kg of bodyweight daily given if necessary in divided doses. All ranges throughout this specification are inclusive. For example from 0.01 to 100 includes the values 0.01 and 100.

Intermittent dosing of the HPPD inhibitor (or of a pharmaceutically acceptable salt thereof) may also be desirable.

In addition to assessment of the overall condition of the patient, the effects of administration of the HPPD inhibitor thereof may be monitored by standard clinical chemical and blood assays.

The invention will now be described by way of the following examples and Figures of which:

Figure 1 is a representation of part of a pathway indicating the metabolism of tyrosine.
Figures 2 to 4 ilustrate the mean catalepsy descent latency data from tables 1.4, 1.5 and 1.6 respectively.

**EXAMPLES**

EXAMPLE 1

Determination of 4-hydroxyphenyl pyruvate dioxygenase (HPPD) enzyme activity *in vitro*

The method used to determine the inhibitory effect of test compounds on HPPD activity was based on the method by Ellis et al 1996 (Ellis, M.K., Whitfield, A.C., Gowans, L.A., Auton, T.R., Provan, W.M., Lock, E.A., Lee, D.L., Smith, L.L. (1996) Characterization of the interaction of 2-[2-nitro-4-(trifluoromethyl)benzoyl]-4,4,6,6-tetramethyl-cyclohexane-1,3,5-trione with rat hepatic 4-hydroxyphenylpyruvate dioxygenase. Chemical Research Toxicology, 9, 24-27).

The principle of the assay is that 4-hydroxyphenyl pyruvate dioxygenase (HPPD), an enzyme that participates in the catabolism of tyrosine, catalyses the oxidative decarboxylation and rearrangement of 4-hydroxyphenylpyruvate (HPPA) to homogentisate, with the incorporation of both atoms of molecular oxygen into the product.

$$HPPA + O_2 \xrightarrow{HPPD} Homogentisate + CO_2$$

Rat liver was homogenised in buffer of the following composition; 0.25 M Sucrose, 5.4 mM EDTA, 20 mM Tris base, pH 7.4, (25% homogenate) using 6 passes of a Potter type homogeniser. The homogenate was then centrifuged at 1,800 g for 10 minutes at 4°C, the pellet was discarded and the supernatant centrifuged at 17,000 g for a further 15 minutes at 4°C. The pellet was then discarded and the supernatant centrifuged at 110,000 g for 80 minutes at 4°C. The supernatant containing the HPPD enzyme from this 110,000 g spin was collected and stored frozen at -70°C and used in the assays of the test compounds (see below).

The effect of the test compound or vehicle (0.04% DMSO) on the formation of homogentisate from HPPA (i.e. HPPD activity), was determined by incubating at 37°C a reaction mixture containing 0.2 M sodium phosphate buffer (pH 7.2), 7.1 μM ascorbate, 0.2 mM HPPA, rat liver cytosol (2.7 mg of protein/ml incubate) and test compound (0 - 300 nM) in a total volume of 4 ml, and measuring the rate of oxygen consumption. Prior to the start of the enzymatic reaction by the addition of HPPA substrate, the enzyme and test compound (inhibitor) were incubated together for 3 minutes. In the absence of inhibitor, the rate of oxygen consumption was 0.96 ± 0.113 μl oxygen/min/mg protein (n=10). The vehicle alone had no effect on HPPD activity.

The rate of oxygen consumption in the presence of the HPPD inhibitor test compound was expressed as a percentage of the rate of oxygen consumption in the absence of the inhibitor, to give a value as a % of the control. In cases where the effects of several concentrations of an inhibitor were investigated, an $IC_{50}$ value (the half-maximal inhibitory concentration of test compound) was determined by plotting the data using a non-linear regression curve-fitting program using a GraphPad Prism™ software package.

The data in Table 1 show the percentage inhibition of HPPD activity at two concentrations of 100 and 300 nM. In addition, $IC_{50}$ values for compound 2 and compound 3.13 are indicated. Data are expressed as mean values from two experiments except where indicated. Where appropriate the values are expressed as the mean ± standard deviation.

Table 1

| Compound No. | % Inhibition at 100nM | % inhibition at 300nM | $IC_{50}$ (nM) |
|---|---|---|---|
| 2 | 64 ± 1.6% (n=6) | 94 ± 0.9% (n=6) | 86 ± 2.4 (n=4) |
| 3.20 | 57% | | |
| 3.21 | 10% | 43% | |
| 3.12 | 40% | | |
| 3.18 | 31% | | |
| 3.22 | 44% | | |
| 3.23 | 24% | | |
| 3.24 | 5% | 34% | |
| 3.11 | 9% | 45% | |
| 3.01 | 8% | 36% | |
| 3.15 | 37% | | |
| 3.25 | 5% | 22% | |
| 3.26 | 20 ± 6.1% (n=4) | 43% | |
| 3.13 | 14% | 41% | 350 (n=1) |

EXAMPLE 2

Plasma tyrosine concentration kinetic profiles in the rat following single oral doses of HPPD inhibitors

The plasma tyrosine concentration kinetic profile in the rat was determined for each of the HPPD inhibitor test compounds by administering a single oral dose to 10-12 week old male Sprague Dawley rats. Compounds 3.20, 3.13 and 3.26 were dosed at 2 mg/kg in 1% carboxymethylcellulose (1% CMC) vehicle, whilst compounds 3.22, 3.12, 3.15, 3.18, 3.23, 3.21, 3.11, 3.01, 3.24 and 3.25 were administered at 10 mg/kg in 1% CMC.

Blood samples to determine plasma tyrosine concentration were obtained at frequent intervals during the first 24 hours post-dose, and at 48 hours post-dose. These samples were either compared with blood samples obtained from control rats which only received an equivalent volume of the vehicle, 1% CMC, or by comparison with control samples obtained from the rats 1 hour prior to dosing with test the compound. Prior to the analysis of the samples for plasma tyrosine concentration, the blood samples were centrifuged at 1800 g for 10 minutes at 4˚C. The plasma was collected and then filtered through a centrifugal micro partition device at 1500 g for 30 minutes at 4˚C. The filtered plasma was then divided into two aliquots and stored frozen at -70˚C for subsequent gradient reverse phase high performance liquid chromatographic (HPLC) analysis of plasma tyrosine. Aliquots of plasma were analysed by HPLC using a 250 x 4.6 mm Hichrom S50DS2 column at 30˚C, eluted with a mobile phase of water / acetonitrile / trifluoroacetic acid (950: 50:2 v/v/v) at a flow rate of 1 ml/min. Detection was by a diode array detector at 274 nm. Appropriate tyrosine standards were run alongside the samples for calibration purposes.

The plasma tyrosine concentration kinetic profile for the test compounds 3.20, 3.13 and 3.26 are shown at the time points 1, 2, 4, 6, 12, 24 and 48 hours post-dose in Table 1.1. The data shown are the mean ± standard deviation (n=4), except for the 6 hour time point where the data were obtained from n=3 rats for the control and 3.26 groups.

Table 1.1

| Time point (hours) | Plasma tyrosine (nmol/ml) | | | |
|---|---|---|---|---|
| | Control | Compound 3.13 2.0 mg/kg | Compound 3.26 2.0 mg/kg | Compound 3.20 2.0 mg/kg |
| 1 | 84 ± 3.3 | 158 ± 6.4 | 173 ± 9.6 | 169 ± 13.6 |
| 2 | 78 ± 5.3 | 209 ± 14.8 | 241 ± 24.0 | 231 ± 30.2 |
| 4 | 72 ± 8.1 | 324 ± 50:2 | 337 ± 53.5 | 353 ± 50.9 |
| 6 | 68 ± 3.8 | 461 ± 52.2 | 504 ± 72.7 | 479 ± 40.0 |
| 12 | 66 ± 8.8 | 801 ± 24.8 | 897 ± 140.8 | 796 ± 44.3 |
| 24 | 86 ± 14.6 | 1281 ± 90.0 | 1987 ± 50.0 | 745 ± 171.8 |
| 48 | 91 ± 6.9 | 115 ± 3.0 | 2449 ± 156.8 | 96 ± 1.7 |

The plasma tyrosine concentration kinetic profile for the test compounds 3.22, 3.12, 3.15, 3.18, 3.23, 3.21, 3.11, 3.01, 3.24 and 3.25 are shown at the time points -1 hour (pre-dose control), 1, 3, 6, 12, 24 and 48 hours post-dose, in Tables 1.2 and 1.3. The data shown are the mean ± standard deviation (n=3), except for the -1 hour time point with compound 3.23, where n=2 (Table 1.2), and for the 3 and 6 hour time points with compound 3.21, where n=2 (Table 1.3).

Table 1.2

| Time (hours) | Plasma Tyrosine Concentration (nmol/ml) | | | | |
|---|---|---|---|---|---|
| | Compound 3.22 | Compound 3.12 | Compound 3.15 | Compound 3.18 | Compound 3.23 |
| -1 | 99 ± 0.8 | 92 ± 3.0 | 100 ± 15.1 | 101 ± 5.1 | 118 |
| 1 | 179 ± 18.5 | 168 ± 20.1 | 177 ± 21.7 | 177 ± 3.5 | 173 ± 22.0 |
| 3 | 319 ± 43.5 | 289 ± 35.6 | 299 ± 18.8 | 303 ± 3.9 | 307 ± 28.0 |
| 6 | 507 ± 86.4 | 451 ± 72.7 | 458 ± 29.4 | 504 ± 29.4 | 502 ± 37.5 |
| 12 | 1011 ± 134.8 | 969 ± 65.2 | 671 ± 119.7 | 1027 ± 62.7 | 1041 ± 88.5 |

(continued)

| Time (hours) | Plasma Tyrosine Concentration (nmol/ml) | | | | |
|---|---|---|---|---|---|
| | Compound 3.22 | Compound 3.12 | Compound 3.15 | Compound 3.18 | Compound 3.23 |
| 24 | 2067 ± 36.6 | 1543 ± 91.1 | 228 ± 76.6 | 1987 ± 133.0 | 1835 ± 444.1 |
| 48 | 2377 ± 518.5 | 114 ± 8.0 | 117 ± 11.3 | 313 ± 216.6 | 299 ± 202.8 |

Table 1.3

| Time (hours) | Plasma Tyrosine Concentration (nmol/ml) | | | | |
|---|---|---|---|---|---|
| | Compound 3.21 | Compound 3.11 | Compound 3.01 | Compound 3.24 | Compound 3.25 |
| -1 | 88 ± 1.6 | 96 ± 7.1 | 89 ± 15.2 | 98 ± 6.5 | 105 ± 7.9 |
| 1 | 177 ± 17.8 | 193 ± 22.9 | 160 ± 7.4 | 184 ± 27.8 | 162 ± 17.7 |
| 3 | 300 | 338 ± 48.9 | 276 ± 12.9 | 313 ± 49.9 | 267 ± 19.4 |
| 6 | 472 | 551 ± 80.7 | 445 ± 32.9 | 548 ± 83.9 | 370 ± 60.8 |
| 12 | 555 ± 39.0 | 1041 ± 106.0 | 666 ± 16.1 | 1008 ± 85.2 | 415 ± 152.8 |
| 24 | 181 ± 23.6 | 2125 ± 65.8 | 264 ± 23.5 | 2110 ± 111.1 | 136 ± 31.2 |
| 48 | 100 ± 6.4 | 993 ± 260.6 | 110 ± 23.9 | 2571 ± 167.7 | 100 ± 13.5 |

EXAMPLE 3

The efficacy of compound 2 and other HPPD inhibitors in the AMPT rat model of Parkinson's disease.

The efficacy of compound 2 and other HPPD inhibitor test compounds in the rat α-methyl-p-tyrosine (AMPT) model of Parkinson's disease was determined by administration of a single oral dose of the test compound to AMPT treated rats. AMPT is a competitive inhibitor of tyrosine hydroxylase. Rats dosed with >150 mg/kg AMPT develop parkinsonian-like behavior and locomotor deficits (e.g. catalepsy and reduced rearing activity) within hours owing to the depletion of striatal dopamine concentration as a consequence of the reduced flux through the dopamine synthetic pathway. Dopamine replacement anti-Parkinson drugs, such as L-3,4-dihydroxyphenylalanine (L-dopa), are effective at restoring normal activity function in this rodent model (Ahlenius, S., Anden, N.E., and Engel, J. (1973). Restoration of locomotor activity in mice by low L-DOPA doses after suppression by alpha-methyltyrosine but not reserpine. Brain Res. 62, 189-199. Ahlenius, S. (1974). Reversal by L-dopa of the suppression of locomotor activity induced by inhibition of tyrosine-hydroxylase and DA-beta-hydroxylase in mice. Brain Res. 69, 57-65. Singh, A., Naidu, P.S., and Kulkarni, S.K. (2003). FK506 as effective adjunct to L-dopa in reserpine-induced catalepsy in rats. Indian J. Exp. Biol. 41, 1264-1268).

Test compound induced reversal of the behavioral deficits induced by AMPT demonstrate efficacy in this animal model for Parkinson's disease.

Male Sprague-Dawley rats (300-350 g) were administered a single i.p. dose of 225 mg/kg AMPT and a single oral dose of 2 mg/kg compound 2. Compound 2 was administered either 16 hours prior to AMPT administration or 4 hours post AMPT dose in order to examine the effects of a large tyrosinaemia (>2000 nmol/mL) and a smaller, sub-maximal tyrosinaemia (<1000 nmol/mL). Behavioral assessment (catalepsy and centre rearing counts) was conducted 8 hours post AMPT administration, this being a time point when the AMPT treated rats were clearly cataleptic. The effect was compared with appropriate vehicle control groups (oral vehicle was 1% carboxymethylcellulose; CMC) and a group that received a 150 mg/kg i.p. dose of L-dopa.

Eight hours after AMPT (or vehicle) administration rats were assessed for catalepsy and centre rearing activity. During the catalepsy test, the front paws of the rat were placed over a horizontal bar suspended 9 cm off the floor. The time taken for the rat to get off the bar (descent latency) was measured with a maximum time of 3 minutes being allowed. Centre rearing counts were determined over a 1 hour monitoring period using an automated activity monitoring system (AM1053). This system used an array of infrared beams to determine the activity and mobility of each animal. Each cage had 48 infrared beams, 24 on each of two levels arranged in an 8 x 16, 1" (25.4 mm) pitch grid. The lower grid measured horizontal x, y movement, whilst the upper grid measured rearing movement. The activity detector operated by counting the number of times the beams changed from unbroken to broken and then incrementing the relevant

counters. Centre rearing counts were incremented when an animal had broken a beam on the upper level and none of the outer two beams were broken, thus detecting rearing when the rats did not use the cage walls for support. The descent latency (seconds) and centre rearing counts for the six treatment groups at 8 hr post AMPT administration are shown in Table 1.4.

Table 1.4

| Group Number | Treatment Group | Mean Catalepsy Descent Latency (s) | Mean Centre Rearing Counts |
|---|---|---|---|
| 1 | AMPT vehicle + compound 2 vehicle | 2 ± 0.1 | 277 ± 61 |
| 2 | AMPT vehicle + compound 2 (+4 hours) | 12 ± 4.3 | 485 ± 108 |
| 3 | AMPT + compound 2 vehicle | 78 ± 11.1 ** | 13 ± 8* |
| 4 | AMPT + compound 2 (-16 hours) | 27 ± 5.4$^{++}$ | 364 ± 74$^{++}$ |
| 5 | AMPT + compound 2 (+4 hours) | 27 ± 6.7$^{++}$ | 298 ± 57$^{++}$ |
| 6 | AMPT + L-dopa (+6.5 hours) | 25 ± 6.9$^{++}$ | 258 ± 71$^{+}$ |
| Data represent the mean ± standard error of the mean for n=12.<br>* p<0.05, ** p<0.01 statistical significance compared to the AMPT vehicle + compound 2 vehicle group (1); +p<0.05, ++ p<0.01 statistical significance compared to the AMPT treated + compound 2 vehicle group (3); one-way ANOVA followed by Dunnett's multiple comparison test. | | | |

Treatment groups were as follows: a vehicle control group (1) was administered AMPT vehicle (saline i.p.) + compound 2 vehicle (1% CMC oral); a compound 2 group (2) was administered AMPT vehicle (saline i.p.) + 2 mg/kg compound 2 oral; an AMPT group (3) was administered 225 mg/kg AMPT i.p. + compound 2 vehicle (1% CMC oral); two groups (4 & 5) were administered a single i.p. dose of 225 mg/kg AMPT and a single oral dose of 2 mg/kg compound 2. In one of these groups (4), compound 2 was administered 16 hour prior to AMPT administration, and in the other group (5), it was administered 4 hour post AMPT dosing. These groups were compared to a positive control group (6) dosed with 225 mg/kg AMPT i.p. + 150 mg/kg L-dopa i.p. 6.5 hour post AMPT dose. L-dopa was administered with a peripheral dopa decarboxylase inhibitor (benserazide), at 100 mg/kg i.p.

Compound 2 substantially reversed the AMPT induced catalepsy and was as effective as 150 mg/kg L-dopa in this respect. Both compound 2 dosing regimens were equally effective, indicating that a sub-maximal tyrosinaemia is sufficient to reverse the effect of AMPT. Compound 2 also reversed the AMPT induced deficits in centre rearing counts as effectively as L-dopa.

Analysis of plasma tyrosine concentration from samples obtained immediately following behavioral assessment confirmed a substantial tyrosinaemia (mean = 2205 ± 238 nmol/mL) in those rats administered compound 216 hours prior to AMPT, and a sub-maximal tyrosinaemia (mean = 345 ± 45 nmol/mL) in those rats which received compound 2, 4 hours post AMPT dose.

In conclusion a single oral dose of 2 mg/kg compound 2 is effective in reversing the AMPT induced catalepsy and centre rearing deficits in the AMPT rat model of Parkinson's disease. These effects occur at time points post dosing when plasma tyrosine concentrations are elevated to >345 nmol/mL. Compound 2 is as effective as L-dopa in this animal model of the disease.

In a second and third series of experiments, the efficacy of eight other HPPD inhibitor test compounds were assessed in the AMPT rat model of Parkinson's disease (Tables 1.5 & 1.6). Male Sprague-Dawley rats (300-350 g) were administered a single i.p. dose of 225 mg/kg AMPT and a single oral dose of 10 mg/kg of the HPPD inhibitor test compound was administered either 1 or 16 hours prior to (-16 or -1 hours) AMPT administration. Behavioral assessment (catalepsy and centre rearing counts) was conducted 8 hours post AMPT administration. The effect was compared with a vehicle control group and a group receiving AMPT alone.

Table 1.5

| Group Number | Treatment Group | Mean Catalepsy Descent Latency (s) | Mean Centre Rearing Counts |
|---|---|---|---|
| 1 | Vehicle Control (saline + 1% CMC) | 2 ± 0.2 | 417 ± 120 |
| 2 | AMPT + 1% CMC | 86 ± 18.5*** | 76 ± 25* |
| 3 | AMPT + compound 3.23 (-16 hours) | 10 ± 3.0$^{+++}$ | 273 ± 63 |

(continued)

| Group Number | Treatment Group | Mean Catalepsy Descent Latency (s) | Mean Centre Rearing Counts |
|---|---|---|---|
| 4 | AMPT + compound 3.18 (-16 hours) | $12 \pm 4.6^{+++}$ | $359 \pm 116^{+}$ |
| 5 | AMPT + compound 3.24 (-16 hours) | $15 \pm 8.9^{+++}$ | $189 \pm 36$ |
| 6 | AMPT + compound 3.11 (-16 hours) | $9 \pm 2.7^{+++}$ | $303 \pm 53$ |

Data represent the mean ± standard error of the mean for n=12.
* p<0.05, *** p<0.001 statistical significance compared to the vehicle control group (1);
$^{+}$p<0.05, $^{+++}$p<0.001 statistical significance compared to the AMPT + 1% CMC group (2); one-way ANOVA followed by Dunnett's multiple comparison test.

Test compounds 3.23, 3.18, 3.24 and 3.11 substantially reversed the AMPT induced catalepsy. The AMPT induced deficits in the mean centre rearing counts were also partially reversed by all the test compounds, although this only reached statistical significance with test compound 3.18. The data demonstrate efficacy of these compounds in this animal model for Parkinson's disease.

Table 1.6

| Group Number | Treatment Group | Mean Catalepsy Descent Latency (s) | Mean Centre Rearing Counts |
|---|---|---|---|
| 1 | Vehicle Control (saline + 1% CMC) | $2 \pm 0.3$ | $529 \pm 71$ |
| 2 | AMPT+ 1% CMC | $131 \pm 16.1$*** | $48 \pm 13$*** |
| 3 | AMPT + compound 3.12 (-16 hours) | $16 \pm 4.2^{+++}$ | $211 \pm 65$ |
| 4 | AMPT + compound 3.20 (-16 hours) | $8 \pm 1.8^{+++}$ | $409 \pm 50^{+++}$ |
| 5 | AMPT + compound 3.01 (-1 hour) | $11 \pm 3.0^{+++}$ | $88 \pm 26$ |
| 6 | AMPT + compound 3.15 (-1 hour) | $23 \pm 8.0^{+++}$ | $199 \pm 54$ |

Data represent the mean ± standard error of the mean for n=12.
*** p<0.001 statistical significance compared to the vehicle control group (1);
$^{+++}$p<0.001 statistical significance compared to the AMPT + 1% CMC group (2); one-way ANOVA followed by Dunnett's multiple comparison test.

Test compounds 3.12, 3.20, 3.01 and 3.15 substantially reversed the AMPT induced catalepsy. The AMPT induced deficits in the mean centre rearing counts were also partially reversed by all the test compounds, although this only reached statistical significance with test compound 3.20. The data demonstrate efficacy of these compounds in this animal model for Parkinson's disease.

In conclusion, a single oral dose of 10 mg/kg of any of the eight HPPD inhibitor test compounds (3.23, 3.18, 3.24, 3.11, 3.12, 3.20, 3.01 and 3.15) is effective in reversing the AMPT induced catalepsy, and either partially or completely reversing the centre rearing deficits in the AMPT rat model of Parkinson's disease. These effects occur at time points when plasma tyrosine concentrations are elevated.

## Claims

1. The use of 2-(2-Nitro-4-Trifluoromethylbenzoyl)-1,3-Cyclohexanedione (compound 2), or a pharmaceutically acceptable salt thereof, in the manufacture of a medicament for use in the treatment of a neurodegenerative disease.

2. The use according to claim 1 wherein said disease is Parkinson's disease.

3. The use according to claim 1 or claim 2 wherein the medicament comprises compound 2 or a pharmaceutically acceptable salt thereof and a further compound which is also capable of inhibiting 4-hydroxyphenylpyruvate dioxygenase (HPPD) in an animal.

4. The use according to any one of the previous claims wherein said medicament comprises a dopamine agonist.

5. The use according to any one of the previous claims wherein said medicament comprises levodopa and a decarboxylase inhibitor.

6. A kit comprising a pharmaceutically effective amount of compound 2 or a pharmaceutically acceptable salt thereof and a pharmaceutically effective amount of a dopamine agonist and a means for the delivery thereof to an animal.

7. A kit comprising a pharmaceutically effective amount of compound 2 or a pharmaceutically acceptable salt thereof and a pharmaceutically effective amount of levodopa and a means for the delivery thereof to an animal.

8. A kit comprising a pharmaceutically effective amount of compound 2 or a pharmaceutically acceptable salt thereof and a pharmaceutically-effective amount of levodopa and a decarboxylase inhibitor and a means for the delivery thereof to an animal.

9. A kit comprising a pharmaceutically effective amount of compound 2 or a pharmaceutically acceptable salt thereof and a pharmaceutically effective amount of a catechol-O-methyl transferase inhibitor and a means for the delivery thereof to an animal.

10. A kit comprising a pharmaceutically effective amount of compound 2 or a pharmaceutically acceptable salt thereof and a pharmaceutically effective amount of a monoamine oxidase inhibitor and a means for the delivery thereof to an animal.

11. A kit comprising a pharmaceutically effective amount of compound 2 or a pharmaceutically acceptable salt thereof and a pharmaceutically effective amount of a further compound which is also capable of inhibiting HPPD in an animal and a means for the delivery thereof to an animal.

12. A pharmaceutical composition comprising as an active ingredient compound 2 or a pharmaceutically acceptable salt thereof and a pharmaceutically effective amount of a further compound which is also capable of inhibiting HPPD in an animal together with a pharmaceutically acceptable diluent or carrier.

13. A pharmaceutical composition comprising a pharmaceutically effective amount of compound 2 or a pharmaceutically acceptable salt thereof and a pharmaceutically effective amount of a dopamine agonist together with a pharmaceutically acceptable diluent or carrier.

14. A pharmaceutical composition comprising a pharmaceutically effective amount of compound 2 or a pharmaceutically acceptable salt thereof and a pharmaceutically effective amount of levodopa together with a pharmaceutically acceptable diluent or carrier.

15. A pharmaceutical composition comprising a pharmaceutically effective amount of compound 2 or a pharmaceutically acceptable salt thereof and a effective amount of levodopa and a decarboxylase inhibitor together with a pharmaceutically acceptable diluent or carrier.

16. A pharmaceutical composition comprising a pharmaceutically effective amount of compound 2 or a pharmaceutically acceptable salt thereof and a pharmaceutically effective amount of a catechol-O-methyl transferase inhibitor together with a pharmaceutically acceptable diluent or carrier.

17. A pharmaceutical composition comprising a pharmaceutically effective amount of a HPPD inhibitor and a pharmaceutically effective amount of a monoamine oxidase inhibitor and a means for the delivery thereof to an animal.

18. A pharmaceutical composition according to any one of claims 12 to 17 which is in a form suitable for oral or parenteral administration.

19. A pharmaceutical composition according to claim 18 which is in palatable form suitable for oral administration selected from the group consisting of: tablets; lozenges; hard capsules; aqueous suspensions; oily suspensions; emulsions; dispersible powders; dispersible granules; syrups and elixirs.

20. A pharmaceutical composition according to claim 18 which is intended for oral use and is in the form of hard or soft

gelatin capsules.

**21.** A pharmaceutical composition as claimed in claim 18 which is in a form suitable for parenteral administration.

**22.** A pharmaceutically effective amount of compound 2 or a composition according to any one of claims 12 to 21 for use in the treatment and/or prevention of a neurodegenerative disease.

**23.** A compound or a composition for the use according to claim 22 wherein said disease is treated.

**24.** A compound or a composition for the use of claim 22 or claim 23 wherein said animal is a human being.

**25.** A compound or a composition for the use according to any one of claims 22 to 24 wherein said neurodegenerative disease is Parkinson’s disease.

**Patentansprüche**

**1.** Verwendung von 2-(2-Nitro-4-trifluormethylbenzoyl)-1,3-cyclohexandion (Verbindung 2) oder eines pharmazeutisch verträglichen Salzes davon bei der Herstellung eines Medikaments zur Verwendung bei der Behandlung einer neurodegenerativen Erkrankung.

**2.** Verwendung gemäß Anspruch 1, wobei die Erkrankung die Parkinsonsche Erkrankung ist.

**3.** Verwendung gemäß Anspruch 1 oder Anspruch 2, wobei das Medikament Verbindung 2 oder ein pharmazeutisch verträgliches Salz davon und eine weitere Verbindung, die ebenfalls zum Hemmen von 4-Hydroxyphenylpyruvatdioxygenase (HPPD) in einem Tier fähig ist, umfasst.

**4.** Verwendung gemäß einem der vorstehenden Ansprüche, wobei das Medikament einen Dopamin-Agonisten umfasst.

**5.** Verwendung gemäß einem der vorstehenden Ansprüche, wobei das Medikament Levodopa und einen Decarboxylasehemmer umfasst.

**6.** Kit, umfassend eine pharmazeutisch wirksame Menge von Verbindung 2 oder eines pharmazeutisch verträglichen Salzes davon und eine pharmazeutisch wirksame Menge eines Dopamin-Agonisten sowie ein Mittel zur Abgabe davon an ein Tier.

**7.** Kit, umfassend eine pharmazeutisch wirksame Menge von Verbindung 2 oder eines pharmazeutisch verträglichen Salzes davon und eine pharmazeutisch wirksame Menge von Levodopa sowie ein Mittel zur Abgabe davon an ein Tier.

**8.** Kit, umfassend eine pharmazeutisch wirksame Menge von Verbindung 2 oder eines pharmazeutisch verträglichen Salzes davon und eine pharmazeutisch wirksame Menge von Levodopa und eines Decarboxylasehemmers sowie ein Mittel zur Abgabe davon an ein Tier.

**9.** Kit, umfassend eine pharmazeutisch wirksame Menge von Verbindung 2 oder eines pharmazeutisch verträglichen Salzes davon und eine pharmazeutisch wirksame Menge eines Catechol-O-methyltransferasehemmers sowie ein Mittel zur Abgabe davon an ein Tier.

**10.** Kit, umfassend eine pharmazeutisch wirksame Menge von Verbindung 2 oder eines pharmazeutisch verträglichen Salzes davon und eine pharmazeutisch wirksame Menge eines Monoaminoxidasehemmers sowie ein Mittel zur Abgabe davon an ein Tier.

**11.** Kit, umfassend eine pharmazeutisch wirksame Menge von Verbindung 2 oder eines pharmazeutisch verträglichen Salzes davon und eine pharmazeutisch wirksame Menge einer weiteren Verbindung, die ebenfalls zum Hemmen von HPPD in einem Tier fähig ist, sowie ein Mittel zur Abgabe davon an ein Tier.

**12.** Pharmazeutische Zusammensetzung, umfassend als Werkstoff Verbindung 2 oder ein pharmazeutisch verträgliches

Salz davon und eine pharmazeutisch wirksame Menge einer weiteren Verbindung, die ebenfalls zum Hemmen von HPPD in einem Tier fähig ist, zusammen mit einem pharmazeutisch verträglichen Verdünnungsmittel oder Träger.

**13.** Pharmazeutische Zusammensetzung, umfassend eine pharmazeutisch wirksame Menge von Verbindung 2 oder eines pharmazeutisch verträglichen Salzes davon und eine pharmazeutisch wirksame Menge eines Dopamin-Agonisten zusammen mit einem pharmazeutisch verträglichen Verdünnungsmittel oder Träger.

**14.** Pharmazeutische Zusammensetzung, umfassend eine pharmazeutisch wirksame Menge von Verbindung 2 oder eines pharmazeutisch verträglichen Salzes davon und eine pharmazeutisch wirksame Menge von Levodopa zusammen mit einem pharmazeutisch verträglichen Verdünnungsmittel oder Träger.

**15.** Pharmazeutische Zusammensetzung, umfassend eine pharmazeutisch wirksame Menge von Verbindung 2 oder eines pharmazeutisch verträglichen Salzes davon und eine pharmazeutisch wirksame Menge von Levodopa und eines Decarboxylasehemmers zusammen mit einem pharmazeutisch verträglichen Verdünnungsmittel oder Träger.

**16.** Pharmazeutische Zusammensetzung, umfassend eine pharmazeutisch wirksame Menge von Verbindung 2 oder eines pharmazeutisch verträglichen Salzes davon und eine pharmazeutisch wirksame Menge eines Catechol-O-methyltransferasehemmers zusammen mit einem pharmazeutisch verträglichen Verdünnungsmittel oder Träger.

**17.** Pharmazeutische Zusammensetzung, umfassend eine pharmazeutisch wirksame Menge eines HPPD-Hemmers und eine pharmazeutisch wirksame Menge eines Monoaminoxidasehemmers sowie ein Mittel zur Abgabe davon an ein Tier.

**18.** Pharmazeutische Zusammensetzung gemäß einem der Ansprüche 12 bis 17, die in einer zur oralen oder parenteralen Verabreichung geeigneten Form vorliegt.

**19.** Pharmazeutische Zusammensetzung gemäß Anspruch 18, die in mundgerechter, zur oralen Verabreichung geeigneter Form vorliegt, ausgewählt aus der Gruppe, bestehend aus Tabletten; Lutschtabletten; Hartkapseln; wässrigen Suspensionen; öligen Suspensionen; Emulsionen; dispergierbaren Pulvern; dispergierbaren Granulatkörnern; Sirupen und Elixieren.

**20.** Pharmazeutische Zusammensetzung gemäß Anspruch 18, die zur oralen Verwendung vorgesehen ist und in der Form von Hart- oder Weichgelatinekapseln vorliegt.

**21.** Pharmazeutische Zusammensetzung gemäß Anspruch 18, die in einer zur parenteralen Verabreichung geeigneten Form vorliegt.

**22.** Pharmazeutisch wirksame Menge von Verbindung 2 oder einer Zusammensetzung gemäß einem der Ansprüche 12 bis 21 zur Verwendung bei der Behandlung und/oder Vorbeugung einer neurodegenerativen Erkrankung.

**23.** Verbindung oder Zusammensetzung zur Verwendung gemäß Anspruch 22, wobei die Erkrankung behandelt wird.

**24.** Verbindung oder Zusammensetzung zur Verwendung gemäß Anspruch 22 oder Anspruch 23, wobei das Tier ein Mensch ist.

**25.** Verbindung oder Zusammensetzung zur Verwendung gemäß einem der Ansprüche 22 bis 24, wobei die neurodegenerative Erkrankung die Parkinsonsche Erkrankung ist.

## Revendications

**1.** Utilisation de la 2-(2-nitro-4-trifluorométhylbenzoyl)-1,3-cyclohexanedione (composé 2), ou un sel pharmaceutiquement acceptable de celle-ci, dans la fabrication d'un médicament pour utilisation dans le traitement d'une maladie neurodégénérative.

**2.** Utilisation selon la revendication 1 dans laquelle ladite maladie est la maladie de Parkinson.

**3.** Utilisation selon la revendication 1 ou la revendication 2 dans laquelle le médicament comprend le composé 2 ou

un sel pharmaceutiquement acceptable de celui-ci et un autre composé qui est également capable d'inhiber la 4-hydroxyphénylpyruvate dioxygénase (HPPD) chez un animal.

**4.** Utilisation selon l'une quelconque des revendications précédentes dans laquelle ledit médicament comprend un agoniste de dopamine.

**5.** Utilisation selon l'une quelconque des revendications précédentes dans laquelle ledit médicament comprend le lévodopa et un inhibiteur de décarboxylase.

**6.** Trousse comprenant une quantité pharmaceutiquement efficace de composé 2 ou un sel pharmaceutiquement acceptable de celui-ci et une quantité pharmaceutiquement efficace d'un agoniste de dopamine et un moyen pour l'administration de ceux-ci à un animal.

**7.** Trousse comprenant une quantité pharmaceutiquement efficace de composé 2 ou un sel pharmaceutiquement acceptable de celui-ci et une quantité pharmaceutiquement efficace de lévodopa et un moyen pour l'administration de ceux-ci à un animal.

**8.** Trousse comprenant une quantité pharmaceutiquement efficace de composé 2 ou un sel pharmaceutiquement acceptable de celui-ci et une quantité pharmaceutiquement efficace de lévodopa et d'un inhibiteur de décarboxylase et un moyen pour l'administration de ceux-ci à un animal.

**9.** Trousse comprenant une quantité pharmaceutiquement efficace de composé 2 ou un sel pharmaceutiquement acceptable de celui-ci et une quantité pharmaceutiquement efficace d'un inhibiteur de catéchol-O-méthyle transférase et un moyen pour l'administration de ceux-ci à un animal.

**10.** Trousse comprenant une quantité pharmaceutiquement efficace de composé 2 ou un sel pharmaceutiquement acceptable de celui-ci et une quantité pharmaceutiquement efficace d'un inhibiteur de monoamine oxydase et un moyen pour l'administration de ceux-ci à un animal.

**11.** Trousse comprenant une quantité pharmaceutiquement efficace de composé 2 ou un sel pharmaceutiquement acceptable de celui-ci et une quantité pharmaceutiquement efficace d'un autre composé qui est également capable d'inhiber HPPD chez un animal et un moyen pour l'administration de ceux-ci à un animal.

**12.** Composition pharmaceutique comprenant en tant que substance active le composé 2 ou un sel pharmaceutiquement acceptable de celui-ci et une quantité pharmaceutiquement efficace d'un autre composé qui est également capable d'inhiber HPPD chez un animal conjointement avec un diluant ou véhicule pharmaceutiquement acceptable.

**13.** Composition pharmaceutique comprenant une quantité pharmaceutiquement efficace de composé 2 ou un sel pharmaceutiquement acceptable de celui-ci et une quantité pharmaceutiquement efficace d'un agoniste de dopamine conjointement avec un diluant ou véhicule pharmaceutiquement acceptable.

**14.** Composition pharmaceutique comprenant une quantité pharmaceutiquement efficace de composé 2 ou un sel pharmaceutiquement acceptable de celui-ci et une quantité pharmaceutiquement efficace de lévodopa conjointement avec un diluant ou véhicule pharmaceutiquement acceptable.

**15.** Composition pharmaceutique comprenant une quantité pharmaceutiquement efficace de composé 2 ou un sel pharmaceutiquement acceptable de celui-ci et une quantité efficace de lévodopa et un inhibiteur de décarboxylase conjointement avec un diluant ou véhicule pharmaceutiquement acceptable.

**16.** Composition pharmaceutique comprenant une quantité pharmaceutiquement efficace de composé 2 ou un sel pharmaceutiquement acceptable de celui-ci et une quantité pharmaceutiquement efficace d'un inhibiteur de catéchol-O-méthyle transférase conjointement avec un diluant ou véhicule pharmaceutiquement acceptable.

**17.** Composition pharmaceutique comprenant une quantité pharmaceutiquement efficace d'un inhibiteur de HPPD et une quantité pharmaceutiquement efficace d'un inhibiteur de monoamine oxydase et un moyen pour l'administration de ceux-ci à un animal.

**18.** Composition pharmaceutique selon l'une quelconque des revendications 12 à 17 qui est sous une forme adaptée

pour administration orale ou parentérale.

**19.** Composition pharmaceutique selon à revendication 18 qui est sous une forme palatable adaptée pour administration orale choisie dans le groupe constitué de : comprimés ; tablettes ; gélules ; suspensions aqueuses ; suspensions huileuses ; émulsions ; poudres dispersibles ; granules dispersibles ; sirops et élixirs.

**20.** Composition pharmaceutique selon la revendication 18 qui est destinée à un usage oral et est sous la forme de gélules ou de capsules.

**21.** Composition pharmaceutique selon la revendication 18 qui est sous une forme adaptée pour administration parentérale.

**22.** Quantité pharmaceutiquement efficace de composé 2 ou composition selon l'une quelconque des revendications 12 à 21 pour utilisation dans le traitement et/ou la prévention d'une maladie neurodégénérative.

**23.** Composé ou composition pour l'utilisation selon la revendication 22 où ladite maladie est traitée.

**24.** Composé ou composition pour l'utilisation de la revendication 22 ou la revendication 23 où ledit animal est un être humain.

**25.** Composé ou composition pour l'utilisation selon l'une quelconque des revendications 22 à 24 où ladite maladie neurodégénérative est la maladie de Parkinson.

**FIGURE 1**

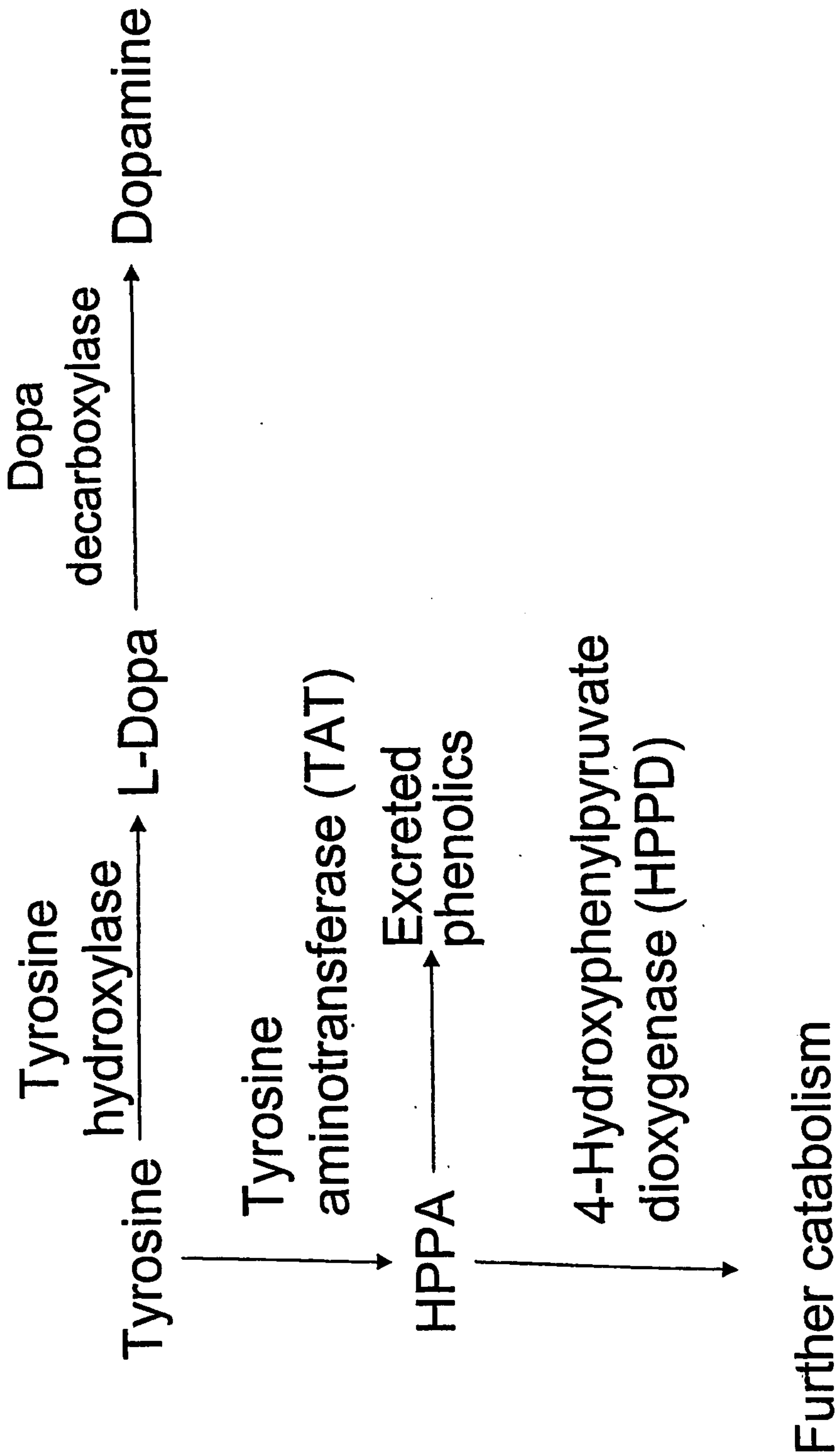
Tyrosine
Tyrosine hydroxylase
L-Dopa
Dopa decarboxylase
Dopamine
Tyrosine aminotransferase (TAT)
HPPA
Excreted phenolics
4-Hydroxyphenylpyruvate dioxygenase (HPPD)
Further catabolism

**FIGURE 2**

TABLE 1.4 - Mean Catalepsy Descent Latency (s)

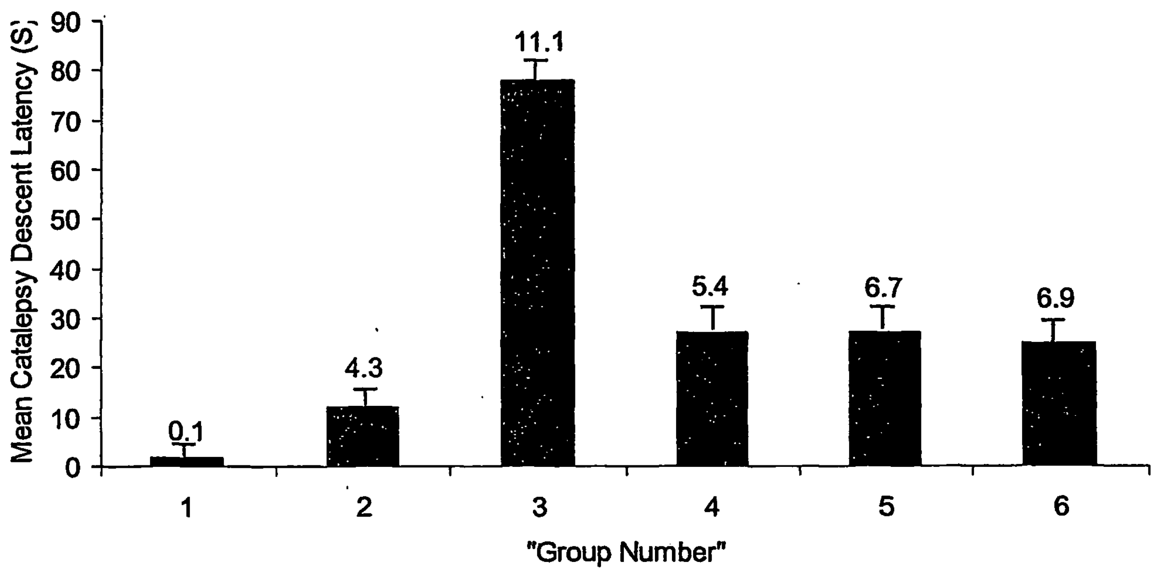

**FIGURE 3**

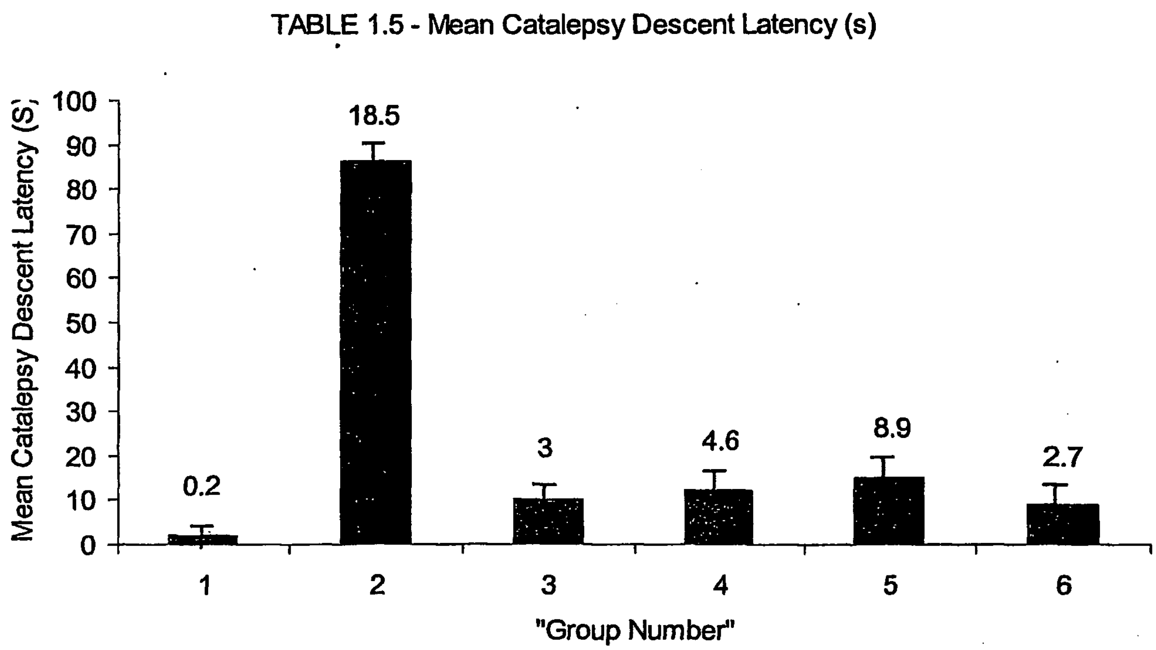
TABLE 1.5 - Mean Catalepsy Descent Latency (s)
Mean Catalepsy Descent Latency (S)
100
90
80
70
60
50
40
30
20
10
0
0.2
18.5
3
4.6
8.9
2.7
1
2
3
4
5
6
"Group Number"

**FIGURE 4**

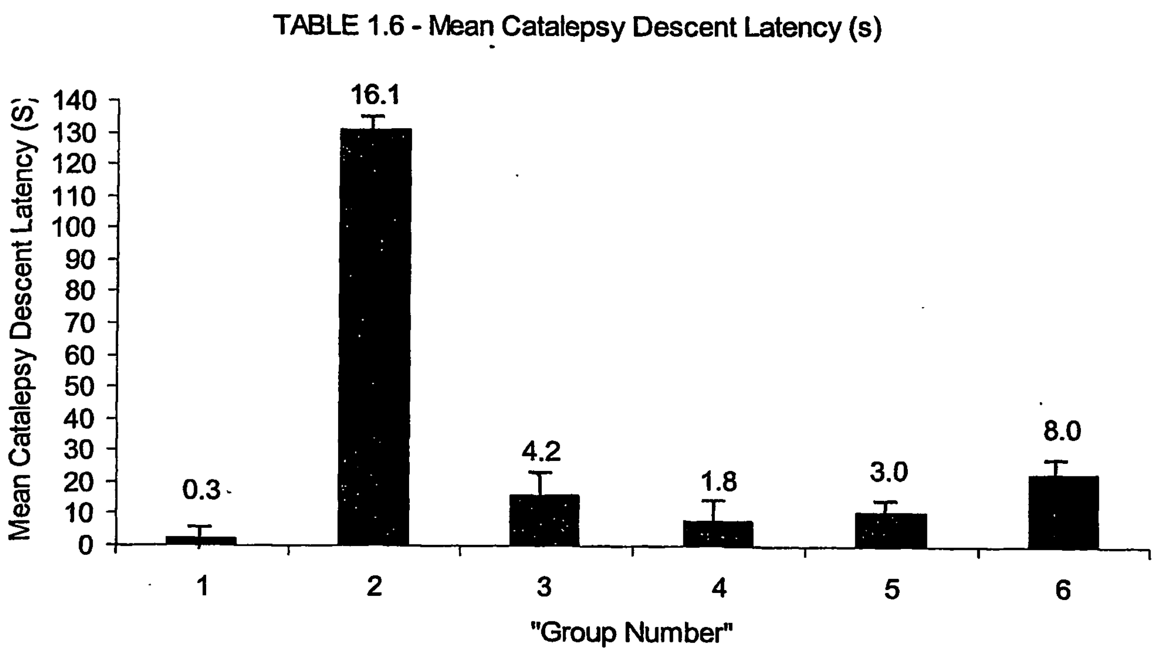
TABLE 1.6 - Mean Catalepsy Descent Latency (s)
Mean Catalepsy Descent Latency (S)
140
130
120
110
100
90
80
70
60
50
40
30
20
10
0
0.3
16.1
4.2
1.8
3.0
8.0
1
2
3
4
5
6
"Group Number"

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0246387 A


### Non-patent literature cited in the description


- Reagents for Organic Synthesis. J Wiley and Sons, 1967, vol. 1, 767-769
- **Haupstein et al.** *J. Amer. Chem. Soc.,* 1954, vol. 76, 1051
- **Ellis, M.K. ; Whitfield, A.C. ; Gowans, L.A. ; Auton, T.R. ; Provan, W.M. ; Lock, E.A. ; Lee, D.L. ; Smith, L.L.** Characterization of the interaction of 2-[2-nitro-4-(trifluoromethyl)benzoyl]-4,4,6,6-tetramethyl-cyclohexane-1,3,5-trione with rat hepatic 4-hydroxyphenylpyruvate dioxygenase. *Chemical Research Toxicology,* 1996, vol. 9, 24-27
- **Ahlenius, S. ; Anden, N.E. ; Engel, J.** Restoration of locomotor activity in mice by low L-DOPA doses after suppression by alpha-methyltyrosine but not reserpine. *Brain Res.,* 1973, vol. 62, 189-199
- **Ahlenius, S.** Reversal by L-dopa of the suppression of locomotor activity induced by inhibition of tyrosine-hydroxylase and DA-beta-hydroxylase in mic. *Brain Res.,* 1974, vol. 69, 57-65
- **Singh, A. ; Naidu, P.S. ; Kulkarni, S.K.** FK506 as effective adjunct to L-dopa in reserpine-induced catalepsy in rats. *Indian J. Exp. Biol.,* 2003, vol. 41, 1264-1268